(19) Europäisches Patentamt
European Patent Office
Office européen des brevets

(11) **EP 2 705 403 B1**

(12) **EUROPÄISCHE PATENTSCHRIFT**

(45) Veröffentlichungstag und Bekanntmachung des Hinweises auf die Patenterteilung:
**01.07.2015 Patentblatt 2015/27**

(21) Anmeldenummer: **12719673.1**

(22) Anmeldetag: **03.05.2012**

(51) Int Cl.:
*A61B 5/00* (2006.01)    *A61F 9/007* (2006.01)
*A61B 3/00* (2006.01)    *A61B 3/10* (2006.01)
*A61F 2/16* (2006.01)

(86) Internationale Anmeldenummer:
**PCT/EP2012/058068**

(87) Internationale Veröffentlichungsnummer:
**WO 2012/150279 (08.11.2012 Gazette 2012/45)**

(54) **VERFAHREN UND VORRICHTUNG ZUR PRÄOPERATIVEN VORHERSAGE EINER POSTOPERATIVEN HORIZONTALEN TIEFENLAGE EINER INTRAOKULARLINSE IN EINEM AUGE**

METHOD AND DEVICE FOR PREOPERATIVELY PREDICTING A POSTOPERATIVE HORIZONTAL DEPTH OF AN INTRAOCULAR LENS IN AN EYE

PROCÉDÉ ET DISPOSITIF POUR PRÉVOIR AVANT L'OPÉRATION UNE POSITION HORIZONTALE EN PROFONDEUR POSTOPÉRATOIRE D'UNE LENTILLE INTRAOCULAIRE DANS UN IL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.05.2011 DE 102011075149**

(43) Veröffentlichungstag der Anmeldung:
**12.03.2014 Patentblatt 2014/11**

(73) Patentinhaber: **Carl Zeiss Meditec AG
07745 Jena (DE)**

(72) Erfinder:
• **BECKER, Ronald
14467 Potsdam (DE)**
• **GERLACH, Mario
16548 Glienicke-Nordbahn (DE)**
• **MÜLLER, Stephanie
16816 Neuruppin (DE)**
• **TSCHIRPKA, Eileen
16515 Oranienburg (DE)**

(74) Vertreter: **Hofstetter, Schurack & Partner
Patent- und Rechtsanwaltskanzlei
PartG mbB
Balanstrasse 57
81541 München (DE)**

(56) Entgegenhaltungen:
WO-A1-2010/028654    WO-A2-2009/059251
US-A1- 2008 231 809    US-A1- 2009 251 664
US-A1- 2011 051 083    US-B1- 7 044 604

• OLSEN T ET AL: "Intraocular lens power calculation with an improved anterior chamber depth prediction algorithm", JOURNAL CATARACT AND REFRACTIVE SURGERY, SURGERY, FAIRFAX, VA, US, Bd. 21, 1. Januar 1995 (1995-01-01), Seiten 313-319, XP009105523, ISSN: 0886-3350

**Beschreibung**

Verfahren und Vorrichtung zur präoperativen Vorhersage einer postoperativen horizontalen Tiefenlage einer Intraokularlinse in einem Auge

**Technisches Gebiet**

Die Erfindung betrifft ein Verfahren zur präoperativen Vorhersage einer postoperativen horizontalen Tiefenlage einer Intraokularlinse in einem Patientenauge, bei welchem Werte von Parametern bereitgestellt werden, wobei die Parameter einen ersten Parameter als Tiefe einer vorderen Augenkammer von Humanaugen, einen zweiten Parameter als horizontale Länge von Humanaugen und einen dritten Parameter als postoperative Lage von Intraokularlinsen in Humanaugen umfassen. Die Erfindung betrifft auch eine Vorrichtung zum Durchführen des Verfahrens.

**Stand der Technik**

Es ist bekannt, dass in einem menschlichen oder tierischen Auge die natürliche Linse entfernt werden kann, insbesondere durch Phakoemulsifikation, und durch eine künstliche Intraokularlinse (IOL) ersetzt werden kann. Die eingesetzte bzw. implantierte Intraokularlinse muss ortsspezifisch jedoch sehr präzise angebracht werden, um die gewünschten optischen Abbildungseigenschaften zu erzielen und den zu korrigierenden Sehfehler des Auges bestmöglich kompensieren zu können. Intraokularlinsen sind dabei in unterschiedlichsten Ausgestaltungen bekannt. Sie weisen üblicherweise einen optischen Teil und daran anschließend einen oder mehrere haptische Teile auf. Mit den haptischen Teilen wird die Intraokularlinse im Auge positionell gehalten.

Um die Korrektur des Sehfehlers bestmöglich durch die Intraokularlinse durchführen zu können, ist diese im Hinblick auf den zu korrigierenden Sehfehler und die Stärke des Sehfehlers positionell und formspezifisch sowie im Hinblick auf die Brechkraft entsprechend auszugestalten.

Dabei ist es bekannt, dass eine entsprechende Intraokularlinse durch Simulation modelliert wird und dann hergestellt und in das Auge implantiert wird.

Aufgrund der Vielzahl von unterschiedlichen Augen im Hinblick auf die Ausgestaltung und Ausmaße, beispielsweise der vorderen Augenkammer, der hinteren Augenkammer, des Kapselsacks, der Dicke der Hornhaut, der Krümmung der Hornhaut, der horizontalen Länge des Auges etc., kann in Verbindung mit dem jeweiligen Sehfehler des spezifischen Auges und der Stärke dieses Sehfehlers eine äußerst hohe Anzahl von unterschiedlichen Situationen entstehen, die hohe Anforderungen an die Modellierung und Herstellung einer Intraokularlinse stellen.

Zur Modellierung derartiger Intraokularlinsen im Hinblick auf die Bestimmung der Brechkraft der Linse sind unterschiedliche Herangehensweisen und Ansätze bekannt. Zum einen sind dort spezifische Augenmodelle hinterlegt, zum anderen werden unterschiedliche Annahmen und Modellrechnungen zugrundegelegt.

Wesentlich ist es für derartige Vorhersagen und Modellierungen, die genaue Lage der Intraokularlinse im zu behandelnden Auge zu kennen, um dann auf dem Weg der simulativen Modellierung die Form und/oder die Brechkraft der benötigten Intraokularlinse exakt bestimmen zu können und auf Basis dieser Modellierung dann die benötigte Intraokularlinse herstellen zu können. Insbesondere ist es dabei dann auch wesentlich, dass die vorhergesagte postoperative Lage der Intraokularlinse im Auge für die Modellierung der Brechkraft der Linse dann nachfolgend auch bestmöglich mit der postoperativen Lage im Auge nach der Implantation übereinstimmt. Nur dann kann der zu korrigierende Sehfehler mit der Intraokularlinse bestmöglich behoben werden.

Die Berechnung der Brechkraft der Linse kann auf Basis unterschiedlicher Ansätze und Berechnungsalgorithmen erfolgen. Wie bereits oben erwähnt, ist dazu wesentlicher Einflussfaktor die Vorhersage und Annahme der postoperativen Lage der Intraokularlinse im Auge. Eine Abweichung dieser vorhergesagten horizontalen Tiefenlage der Intraokularlinse von der dann tatsächlich vorhandenen Tiefenlage der Intraokularlinse im Auge nach der Operation ist ein wesentlicher Haupteinflussfaktor dafür, ob das Operationsergebnis und die daraus resultierende Sehfähigkeit des Auges als erfolgreich beurteilt werden kann oder nicht.

Zur Bestimmung einer ELP (Estimated Lens Position) als postoperativer Vorhersagewert sind verschiedene Ansätze bekannt. Beispielsweise seien hier Ansätze nach der SRK-Methode, Ansätze und Berechnungsweisen nach Binkhorst, nach Shammas, nach Holladay oder nach Haigis genannt.

Diese Aufzählung ist nicht abschließend und es sind auch darüber hinaus weitere Modellberechnungen bekannt.

Bei einigen Modellen wird dabei in vereinfachter Weise ein dreidimensionales Koordinatensystem, welches durch die Parameter der präoperativen Tiefe der vorderen Augenkammer eines Auges, der horizontalen Länge eines Auges und einer postoperativen horizontalen Lage der Intraokularlinse im Auge erzeugt wird, zu Grunde gelegt. In diesem dreidimensionalen Koordinatensystem, beispielsweise bei dem Ansatz von Haigis, wird dann eine vollständige Ebene erzeugt. Diese in dem dreidimensionalen Raum angeordnete Ebene ermöglicht dann, abhängig von den präoperativen Werten der Kammertiefe und der Augenlänge den postoperativen Wert der Lage der Intraokularlinse im Auge vorherzusagen. Aufgrund der starken Vereinfachungen und Annahmen liefern diese Ansätze zu ungenaue Vorhersagen. Diese wirken sich dann einerseits auf die Modellierung zur Bestimmung der Brechkraft der Linse aus, was sich dann wiederum verstärkt negativ auf die tatsächliche Korrekturwirkung der modellierten und hergestellten Intraokularlinse im natürlichen Auge auswirkt. Wird zunächst bei der Modellierung somit die vorhergesagte postoperative horizontale Tiefenlage der Intraokularlinse zu ungenau gemacht, hat dies einen wesentlichen Einfluss auf das Ergebnis des Sehvermögens nach der Implantation.

Aus der US 5,282,852 ist ein Verfahren zur Berechnung der Brechkraft einer Intraokularlinse bekannt. Darüber hinaus ist aus der US 2009/251664 A1 ebenfalls ein Verfahren sowie ein System zur Bestimmung der Brechkraft einer Intraokularlinse bekannt.

Darüber hinaus ist aus der DE 696 30 544 T2 ein Verfahren zum präoperativen Auswählen einer Intraokularlinse, die in ein Auge implantiert werden soll, bekannt.

## Darstellung der Erfindung

Es ist Aufgabe der vorliegenden Erfindung, ein Verfahren und eine Vorrichtung zu schaffen, mit welchem die Vorhersage einer postoperativen Tiefenlage einer Intraokularlinse im Auge verbessert werden kann.

Diese Aufgabe wird durch ein Verfahren und eine Vorrichtung gemäß den unabhängigen Ansprüchen aufgelöst.

Bei einem erfindungsgemäßen Verfahren zur präoperativen Vorhersage einer postoperativen horizontalen Tiefenlage einer Intraokularlinse in einem ersten zu behandelnden Auge bzw. einem Patientenauge, in dem die natürliche Linse durch eine Intraokularlinse ersetzt werden soll, werden Werte von Parametern bereitgestellt, wobei die Parameter einen ersten Parameter als Tiefe einer vorderen Augenkammer von Humanaugen, einen zweiten Parameter als horizontale Länge von Humanaugen und einen dritten Parameter als postoperative Lage von Intraokularlinsen in Humanaugen umfassen. Aus diesen Werten werden spezifische Wertetripel mit jeweils einem Wert der drei genannten Parameter als Haupt-Stützstellen ausgewählt und die Haupt-Stützstellen werden in ein durch die drei Parameter aufgespanntes dreidimensionales Koordinatensystem eingetragen. Die Haupt-Stützstellen werden durch Rand-Verbindungsgeraden verbunden und zumindest durch die Lagen der Rand-Verbindungsgeraden wird ein im dreidimensionalen Koordinatensystem uneben geformtes Vorhersagenetz zur Vorhersage der postoperativen horizontale Tiefenlage der Intraokularlinse im Patientenauge erzeugt. Die Humanaugen sind nicht das Patientenauge.

Zumindest durch die Lagen der Rand-Verbindungsgeraden wird ein im dreidimensionalen Koordinatensystem uneben geformtes und simulativ gebildetes Vorhersagenetz erzeugt, wobei auf Basis dieses Vorhersagenetzes eine Vorhersage der postoperativen Tiefenlage der Intraokularlinse im ersten Auge erfolgt.

Insbesondere abhängig von den gemessenen Parametern des Patientenauges, insbesondere der Tiefe der vorderen Augenkammer und der Länge des Patientenauges, und von dem Vorhersagenetz wird die postoperative horizontale Tiefenlage bestimmt und als Information bereitgestellt.

Mit dem erfindungsgemäßen Verfahren wird somit ermöglicht, dass vor dem chirurgischen Eingriff am Patientenauge zur Implantation einer Intraokularlinse simulativ vorhergesagt werden kann, wo die Intraokularlinse nach dem Implantieren im Patientenauge positionell im Hinblick auf die horizontale Tiefenlage angeordnet sein wird. Durch die spezifische Vorgehensweise einer Erzeugung von Haupt-Stützstellen und insbesondere die ganz spezifische Verbindung dieser Stützstellen durch Rand-Verbindungsgeraden wird in besonderer Vorteilhaftigkeit ein unebenes Vorhersagenetz erzeugt. Mit diesem kann die Präzision der Vorhersage dieser postoperativen horizontalen Tiefenlage wesentlich verbessert werden. Da diese postoperative horizontale Tiefenlage auch wesentlicher und entscheidender Einflussparameter für die Berechnung und Modellierung der Intraokularlinse im Hinblick auf die erforderliche Brechkraft ist, kann gerade im Zusammenspiel mit der präziseren Vorhersage dieser postoperativen horizontalen Tiefenlage auch die Modellierung der Linse im Hinblick auf ihre Form und ihre Brechkraft präzisiert werden. In Kombination dieser beiden präziser modellierbaren und vorhersagbaren Parameter kann somit dann auch im nachfolgenden operativen Eingriff eine bestmögliche Korrektur des Sehfehlers durch die Intraokularlinse erzielt werden. Unerwünschte Abweichungen von der präoperativen Vorhersage der postoperativen horizontalen Tiefenlage und der dann nachfolgend tatsächlichen horizontalen Tiefenlage der Intraokularlinse im Auge nach dem chirurgischen Eingriff können dadurch vermieden werden.

Durch die ganz spezifische Gestaltung des Vorhersagenetzes mit der ganz spezifischen Form im Hinblick auf eine unebene Formgebung im dreidimensionalen Raum kann den unterschiedlichsten Augenausgestaltungen und den damit verbundenen unterschiedlichsten Anforderungen im Hinblick auf die Bestimmung dieser Tiefenlage verbessert Rechnung getragen werden. Insbesondere unübliche oder nicht gängige Ausgestaltungen von Patientenaugen im Hinblick auf die genannten Parameter der Tiefe der vorderen Augenkammer und der Länge des Auges können dadurch wesentlich besser berücksichtigt werden. Insbesondere bei diesbezüglich mit geringerer Häufigkeit auftretenden Augenformen und Ausmaßen kann somit die Vorhersage einer postoperativen horizontalen Tiefenlage wesentlich präzisiert werden. Dadurch kann auch im Nachfolgenden die erforderliche Modellierung der Intraokularlinse im Hinblick auf ihre Brechkraft verbessert und präzisiert werden und somit insbesondere für derartig eher selten vorkommende Augen das Ergebnis eines chirurgischen Eingriffs wesentlich verbessert werden.

Insbesondere bezeichnet der Parameter der horizontalen Tiefenlage der Intraokularlinse im Patientenauge die Wegstrecke auf der horizontalen optischen Hauptachse des Auges zwischen der Hornhaut, insbesondere der Vorderseite der Hornhaut, und der vertikalen Mittenebene bzw. der vertikalen Äquatorebene, nachfolgend als vertikaler Äquator bezeichnet, der Intraokularlinse im Auge.

Es kann vorgesehen sein, dass Werte der anderen Humanaugen, die die Datenbasis für die Erzeugung und Auswahl der Wertetripel als Haupt-Stützstellen darstellen, gemessen werden. Es kann vorgesehen sein, dass dabei bei den anderen Humanaugen Werte von Tiefen von vorderen Augenkammern und Werte von horizontalen Längen dieser anderen Humanaugen gemessen werden. Insbesondere wird dann nach dem chirurgischen Eingriff an diesen anderen Humanaugen postoperativ der Wert von Tiefenlagen von implantierten Intraokularlinsen gemessen. Insbesondere wird hier der Wert bis zur Vorderfläche der Linse gemessen. Dadurch kann eine sehr große und umfängliche Datenbasis generiert werden.

Insbesondere wird zu diesem Wert dazu dann die Hälfte der Dicke der bekannten eingesetzten IOL addiert, um einen zur horizontalen Tiefenlage vergleichbaren Wert zu erhalten. Dadurch wird die Genauigkeit nochmals erhöht.

Vorzugsweise wird vorgesehen, dass Werte, die die Datenbasis für die Haupt-Stützstellen bilden, teilweise gemessen und teilweise geschätzt werden. Insbesondere wird vorgesehen, dass die Werte für die Vorderkammertiefe der anderen Humanaugen, die die Datenbasis bilden, gemessen werden. Insbesondere werden Werte für die Länge der anderen Augen, die die Datenbasis bilden, gemessen. Vorzugsweise werden Werte von postoperativen Tiefenlagen der Intraokularlinsen von Humanaugen, die die Datenbasis für die Haupt-Stützstellen bilden, geschätzt und/oder berechnet. Insbesondere können diese Werte auch an den anderen Humanaugen gemessen werden.

Es kann auch vorgesehen sein, dass alle Werte der Haupt-Stützstellen geschätzt und/oder berechnet werden. Der Aufwand für ein Messen entfällt dann.

Besonders vorteilhaft ist es, wenn abhängig davon, welche Haupt-Stützstelle gebildet werden sollen, dann geschätzte und/oder gemessene und/oder berechnete Werte für den Parameter der Vorderkammertiefe berücksichtigt werden. Die Genauigkeit des Vorhersagenetzes wird dadurch erhöht.

Insbesondere kann durch redundante Wertbildung, insbesondere durch unterschiedliche Art einer Wertbildung, die Genauigkeit erhöht und eine Plausibilitätsprüfung erfolgen.

Vorzugsweise werden für Haupt-Stützstellen, welche randseitig das Vorhersagenetz begrenzen, sowohl geschätzte als auch gemessene Werte der horizontalen Tiefenlage berücksichtigt.

Die Auswahl von Haupt-Stützstellen kann somit in vielfältiger und variabler Weise erfolgen, sodass auch hier eine große Flexibilität erreicht ist. Dadurch kann auch die Erzeugung der Rand-Verbindungsgeraden und das resultierende Vorhersagenetz in vielfältiger Weise gestaltet und angepasst werden.

Durch diese Variabilität und Flexibilität kann den jeweiligen individuellen Situationen Rechnung getragen werden und eine sehr dynamische Vorgehensweise bei der Erstellung von Informationen für die präoperative Vorhersage dieser Tiefenlage erreicht werden.

Das Vorhersagenetz ist ein definiert und individuell erzeugtes Gebilde als Aussageskelett für die zu bestimmende Tiefenlage der Intraokularlinse. Sie stellt daher ein darstellbares Hilfswerkzeug dar, anhand dem die Vorhersage der Tiefenlage nachvollziehbar und besonders präzise ermöglicht ist. Die Skelettform wird durch ganz definiert festgelegte und gebildete Stützpunkte charakterisiert. Diese werden darüber hinaus mit definierten geometrischen Elementen, nämlich Geraden, verbunden. Im Hinblick auf Aussagepräzision und Bestimmungsaufwand hat dies besondere Vorteile.

Vorzugsweise wird vorgesehen, dass die präoperativ vorherzusagende postoperative Tiefenlage der Intraokularlinse im Patientenauge abhängig von zumindest einer zur Erzeugung des Vorhersagenetzes beitragenden Verbindungsgeraden bestimmt wird. Dies ist besonders vorteilhaft, da dadurch die Aussage über die postoperative horizontale Tiefenlage präzisiert werden kann. Dies dahingehend, da durch das Vorhersagenetz bereits ein Konstrukt aufgespannt ist, das durch die unebene Formgebung den unterschiedlichen Augenmaßen und Werten besonders Rechnung trägt und sich entsprechend annähert, wobei dann durch die beitragenden Verbindungsgeraden eine besonders aussagekräftige Bestimmung ermöglicht ist. Dies, da durch die Verbindungsgeraden das Vorhersagenetz quasi aufgespannt wird und diese Verbindungsgeraden somit quasi von vornherein die Haupteinflussfaktoren für die Berechnung zur präoperativen Vorhersage der Tiefenlage darstellen.

Vorzugsweise wird vorgesehen, dass das Vorhersagenetz aus einer Mehrzahl von Teilmaschen erzeugt wird, die somit insbesondere Teilnetze darstellen.

Die Haupt-Stützstellen werden vorzugsweise so gewählt, dass sie jeweils Eckpunkte zumindest einer Teilmasche sind. Durch eine derartige Ausgestaltung des Vorhersagenetzes kann die unebene Formgebung im Hinblick auf eine präzisere Vorhersage einer postoperativen horizontalen Tiefenlage einer Intraokularlinse abhängig von dem Vorhersagenetz präzisiert werden. Indem die Haupt-Stützstellen die Eckpunkte der Teilmaschen darstellen, kann die Spezifizierung der Form der Teilmaschen, die Größe und Lage im dreidimensionalen Koordinatensystem individuell festgelegt werden. Die Vorhersagepräzision lässt sich dadurch nochmals steigern, da die Teilmaschenbestimmung auch wiederum sehr variabel und flexibel erfolgen kann und sehr situationsangepasst festgelegt werden kann. Indem die Stützstellen die Eckpunkte von Teilmaschen darstellen kann gerade im Zusammenhang mit der Vorgehensweise, dass diese Haupt-Stützstellen durch Rand-Verbindungsgeraden verbunden werden, die Vorgehensweise bei der Berechnung der postoperativen Tiefenlage exakter erfolgen. Die Zuordnung von berechneten Werten zu spezifizierten Teilmaschen, die dann auch im Hinblick auf andere Teilmaschen des Vorhersagenetzes lagemäßig und/oder größenmäßig identifiziert werden können, ist dann einfacher und aufwandsärmer möglich. Dennoch ist die Aussagepräzision nicht beeinträchtigt.

Vorzugsweise ist vorgesehen, dass die Teilmaschen des Vorhersagenetzes als Rechtecke aufgespannt werden. Indem alle Teilmaschen die gleiche Formgebung haben und somit vier Eckpunkte aufweisen, die durch jeweils vier Haupt-Stützstellen gebildet werden, kann ein vollständig zusammenhängendes Vorhersagenetz konstruiert werden. Gerade Rechtecke ermöglichen dabei, Rand-Verbindungsgeraden einfach zu erzeugen und im Hinblick auf eine Projektion in eine Ebene diese parallel verlaufen zu lassen. Die weitere Berechnung für eine präoperative Vorhersage der Tiefenlage der Intraokularlinse ist dadurch besonders einfach und aufwandsarm und dennoch äußerst präzise möglich.

Vorzugsweise wird vorgesehen, dass die Teilmaschen des sich dreidimensional ausbildenden flächigen Vorhersagenetzes in eine Projektionsebene bzw. Koordinatenebene übertragen werden, wobei die Koordinatenebene durch die senkrecht zueinander stehenden Koordinatenachsen für die Parameter der Tiefe von vorderen Augenkammern und der Länge von Augen aufgespannt wird.

Insbesondere wird dann in der Koordinatenebene eine Rasterung durchgeführt, wobei dazu die Teilmaschen des Vorhersagenetzes als Quadranten dieser Rasterung zugrunde gelegt werden.

Zur Vorhersage der Tiefenlage werden dann Messwerte des Patientenauges im Hinblick auf die Tiefe der vorderen Augenkammer des Patientenauges und der Länge des Patientenauges mit den Werten der Quadranten verglichen und daraus bestimmt, in welchem Quadranten das Patientenauge klassifiziert wird. Insbesondere wird abhängig von den Rand-Verbindungsgeraden diese Quadranten dann die vorherzusagende Tiefenlage der Intraokularlinse im Auge bestimmt.

Vorzugsweise ist vorgesehen, dass die Rechtecke so in das Koordinatensystem eingebracht werden, dass die Rand-Verbindungsgeraden zwischen jeweils zwei Eckpunkten in einer Teilmasche bei einer Projektion der Rand-Verbindungsgeraden in die Koordinatenebene, welche durch die senkrecht zueinander stehenden Koordinatenachsen für die Parameter der Tiefe von vorderen Augenkammern und der Länge von Augen aufgespannt wird, parallel zu den Koordinatenachsen verlaufen. Dies ist besonders hervorzuheben, da dadurch ein sehr differenziertes und aussagekräftiges Vorhersagenetz gebildet werden kann. Die Abdeckung von sehr vielen unterschiedlichen Wertepaarkombinationen bezüglich der Parameter der Tiefe von vorderen Augenkammern und der Länge von Augen kann dadurch abgedeckt werden. Nicht zuletzt kann durch eine derartige spezifische Lageanordnung der Rand-Verbindungsgeraden die Berechnung der postoperativen Lage der Intraokularlinse und somit den Wert des dritten Parameters in diesem dreidimensionalen Koordinatensystem sehr vereinfacht und im Aufwand reduziert werden. Die Bestimmung der präoperativen Vorhersage kann dadurch auch sehr schnell erfolgen. Dies hat insbesondere wesentliche Vorteile auf den Rechenaufwand einerseits und die schnell bereitzustellenden Informationen andererseits.

Vorzugsweise wird vorgesehen, dass die Hauptstützstellen so ausgewählt werden, dass die Längen der in die Koordinatenebene projizierten Rand-Verbindungsgeraden der Teilflächen in der Koordinatenebene in Richtung der ersten Koordinatenachse gleich erzeugt werden. Dadurch kann eine ganz spezifische Segmentierung bzw. Quadrantenbildung erfolgen.

Vorzugsweise ist dabei vorgesehen, dass auf der ersten Koordinatenachse als Parameter die Tiefe der vorderen Augenkammer aufgetragen ist. Gerade für diesen Parameter lassen sich dann gleich lange Teilmaschen erzeugen, sodass die jeweils darunter fallenden bzw. umfassten tatsächlich gemessenen Werte von Augen auch entsprechend eingruppiert werden können. Indem im Hinblick auf diese Koordinatenachse die Längen gleichgesetzt werden, kann auch bezüglich der verfügbaren Anzahl von bereits vorliegenden Daten der anderen Humanaugen eine Aussage über die Häufigkeit von derartigen Werten in den jeweiligen Quadranten ermöglicht werden.

Vorzugsweise ist vorgesehen, dass die Längen der in die Koordinatenebene projizierten Rand-Verbindungsgeraden der Teilflächen in der Koordinatenebene in Richtung der ersten Koordinatenachse unterschiedlich erzeugt werden. Damit kann in besonders vorteilhafter Weise eine den jeweils für die einzelnen Häufigkeiten der vorliegenden Werte angepasste Längendimensionierung ermöglicht werden. Die Aussagepräzision kann dadurch gesteigert werden, da gerade dann, wenn eine Vielzahl von Werten für einen ganz spezifischen Quadrantenbereich vorliegen, eine entsprechende Längengestaltung noch genauer erfolgen kann.

Vorzugsweise ist vorgesehen, dass zumindest drei derartige Teillängen entlang der ersten Koordinatenachse gebildet werden, sodass zumindest entlang dieser Koordinatenachse vier Haupt-Stützstellen gebildet sind. Vorzugsweise ist dabei vorgesehen, dass die erste Haupt-Stützstelle bei einem Wert der Tiefe der vorderen Augenkammer bei 1,50 mm liegt. Vorzugsweise liegt eine zweite Haupt-Stützstelle bei einem gleichen Wert der Länge des Auges wie die erste Haupt-Stützstelle bei einem Wert 2,90 mm bezüglich der Tiefe der vorderen Augenkammer. Vorzugsweise ist vorgesehen, dass eine dritte Haupt-Stützstelle bei einem gleichen Wert der Länge des Auges wie die beiden ersten Haupt-Stützstellen bei einem Wert 3,40 mm bezüglich der Tiefe der vorderen Augenkammer liegt. Insbesondere ist vorgesehen, dass eine vierte Haupt-Stützstelle beim gleichen Wert der Länge des Auges, wie ihn die drei ersten Haupt-Stützstellen aufweisen, bei einem Wert von 6,00 mm bezüglich der Tiefe der vorderen Augenkammer liegt.

Vorzugsweise ist vorgesehen, dass die Längen der in die Koordinatenebene projizierten Rand-Verbindungsgeraden von zumindest zwei Teilmaschen in der Koordinatenebene in Richtung der zweiten Koordinatenachse unterschiedlich erzeugt werden. Insbesondere ist auf der zweiten Koordinatenachse als Parameter die Länge des Auges aufgetragen. Im Hinblick auf die unterschiedliche Längengestaltung ergeben sich die analogen Vorteile, wie sie bereits oben zur unterschiedlichen Längengestaltung der Teilmaschen in Richtung der ersten Koordinatenachse genannt wurden.

Vorzugsweise ist vorgesehen, dass auch entlang der zweiten Koordinatenachse eine Mehrzahl von weiteren Haupt-Stützstellen aufgetragen ist, die allesamt auf dem gleichen Wert des Parameters bezüglich der Tiefe der vorderen Augenkammer beruhen. Sie variieren somit lediglich im Hinblick auf den Wert der Länge eines Auges. Die diesbezüglich getroffenen Aussagen beziehen sich allesamt auf die Situation, wenn die Haupt-Stützstellen und die Rand-Verbindungsgeraden in die Koordinatenebene projiziert werden, die durch die beiden Koordinatenachsen aufgespannt wird, auf denen einerseits die Tiefe der vorderen Augenkammer und andererseits die Länge des Auges aufgetragen ist.

Vorzugsweise wird vorgesehen, dass die bereits oben genannte erste Haupt-Stützstelle bezüglich ihres Werts in Richtung der zweiten Koordinatenachse bei einem Wert 15,00 mm angeordnet ist. Insbesondere ist eine weitere Haupt-Stützstelle bei einem Wert 22,50 mm bezüglich der Länge des Auges angeordnet. Eine weitere Haupt-Stützstelle ist insbesondere bei einem Wert 25,00 mm der Länge des Auges aufgetragen und eine weitere Haupt-Stützstelle ist bei einem Wert 40,00 mm der Länge des Auges aufgetragen.

Vorzugsweise wird vorgesehen, dass die Länge einer in die Koordinatenebene projizierten Rand-Verbindungsgeraden in Richtung zumindest einer Koordinatenachse abhängig von der Anzahl der vorhandenen präoperativen Werte des auf dieser Koordinatenachse aufgetragenen Parameters erzeugt wird.

Insbesondere wird vorgesehen, dass diese Länge umso kleiner gewählt wird, je größer die Anzahl der präoperativen Werte ist. Die Vorhersagegenauigkeit lässt sich dadurch verbessern.

Vorzugsweise ist vorgesehen, dass das Vorhersagenetz matrixartig aufgebaut wird und mit zumindest neun Teilmaschen erzeugt wird. Insbesondere wird zumindest eine 3x3-Matrix erzeugt.

Vorzugsweise wird abhängig von dem Wertepaar der Tiefe der vorderen Augenkammer und der Länge des Patientenauges, welche Werte gemessen werden, diejenige Teilmasche des Vorhersagenetzes ausgewählt, durch welche das Wertepaar in den Koordinatenachsen umfasst wird.

Im Hinblick auf die Bestimmung der Werte der postoperativen horizontalen Tiefenlagen der Intraokularlinsen in den anderen Humanaugen werden einerseits Messungen berücksichtigt. Zusätzlich oder anstatt dazu können für diese Werte der Haupt-Stützstellen in dieser dritten Raumrichtung Berechnungen oder Schätzungen von Werten berücksichtigt werden. Dadurch kann ein Vergleich und eine präzisere Bestimmung dieser Werte erfolgen, so dass auch die Haupt-Stützstellen bereits sehr genau sind und eine sehr präzise Position einnehmen. Dadurch wird auch die Präzision bezüglich der Lage der Rand-Verbindungsgeraden verbessert, was sich wiederum auf die Präzision der unebenen Formgebung des Vorhersagenetzes auswirkt, um dann abhängig davon eine besonders präzise präoperative Vorhersage einer postoperativen horizontalen Tiefenlage der Intraokularlinse im Patientenauge, welches das nachfolgend chirurgisch zu behandelnde Auge darstellt, gewährleisten zu können. Durch die Schaffung der Möglichkeit einer uneben im Raum sich gestaltenden Vorhersagefläche auf Basis des uneben ausgestalteten Vorhersagenetzes können sehr individuell gestaltete Freiformen dieses Vorhersagenetzes resultieren, was jedoch exakt der präoperativen Aussage über die Lage der Intraokularlinse im Patientenauge positiv zugute kommt.

Vorzugsweise wird vorgesehen, dass für den Wert der Tiefe der vorderen Augenkammer des Patientenauges eine erste zur Erzeugung des Vorhersagenetzes beitragende Zwischen-Verbindungsgerade abhängig von den Werten der Rand-Verbindungsgeraden der ausgewählten Teilmasche an der Stelle des Werts der Tiefe der vorderen Augenkammer bestimmt wird. Zusätzlich oder anstatt dazu kann auch vorgesehen sein, dass für den Wert der Länge des Patientenauges eine zweite zur Erzeugung eines Vorhersagenetzes beitragende Zwischen-Verbindungsgerade abhängig von den Werten der Rand-Verbindungsgeraden der ausgewählten Teilfläche an der Stelle des Werts der Länge des Patientenauges bestimmt wird. Es wird also ermöglicht, dass dann, wenn spezifisch gemessene Werte des Patientenauges bezüglich der Tiefe der vorderen Augenkammer und der Länge des Auges, die dann nicht explizit auf einer Haupt-Stützstelle, wie sie ausgewählt wurden, liegen, dennoch im Weiteren eine sehr präzise präoperative Vorhersage der horizontalen Tiefenlage der Intraokularlinse im Patientenauge durch entsprechende Berechnung erfolgen kann.

Indem die Rand-Verbindungsgeraden durch exakte lineare Gleichungen beschrieben werden können und diese insbesondere bei einer Projektion in die Koordinatenebene aufgespannt durch die oben genannten Parameter der Tiefe der vorderen Augenkammer und der Länge des Auges jeweils parallel dann zu den jeweiligen Koordinatenachsen verlaufen, kann sehr präzise dann auch die Berechnung der horizontalen Tiefenlage des Patientenauges erfolgen. So kann nämlich nach Feststellung, in welchem Quadranten bzw. durch welche Teilmasche die gemessenen Werte des Patientenauges erfasst werden, dann auch ganz spezifisch auf die diese Teilmasche einrahmenden Rand-Verbindungsgeraden zurückgegriffen werden, die bereits vorab durch Verbindung der entsprechenden Haupt-Stützstellen ermittelt wurden. Werden dort in diese Rand-Verbindungsgeraden dann die gemessenen Werte der Tiefe der vorderen Augenkammer des Patientenauges und der Länge des Patientenauges eingesetzt, so können auf den Rand-Verbindungsgeraden jeweils spezifische Zwischenwerte für die Tiefenlage der Intraokularlinse bestimmt werden. Da insbesondere bei einer Erzeugung einer Teilfläche in Form eines Rechtecks bei Projektion in die Koordinatenebene aufgespannt durch die Parameter der Tiefe der vorderen Augenkammer und der Länge des Auges jeweils zwei gegenüberliegende Rand-Verbindungsgeraden vorliegen, entstehen bei dem konkreten Einsetzen der bekannten gemessenen Werte der Tiefe der vorderen Augenkammer des Patientenauges und der Länge des Patientenauges jeweils wiederum zwei Zwischen-Stützstellen auf den jeweiligen Rand-Verbindungsgeraden, die dann wiederum individuell zur Erzeugung der Zwischen-Verbindungsgeraden verbunden werden können.

Vorzugsweise ergeben sich dann für alle gemessenen Wertepaare der Tiefe der vorderen Augenkammer des Patientenauges und der Länge des Patientenauges entsprechende Werte für eine postoperative horizontale Tiefenlage eine Intraokularlinse in dem Patientenauge, die sich dann aus zumindest einer Zwischen-Verbindungsgeraden berechnen lässt.

Es kann dabei vorgesehen sein, dass die Teilmaschen zwischen den Rand-Verbindungsgeraden und dazu die gebildeten Teilflächen eben ausgebildet sind und geneigt im Raum orientiert sind. Bei einer derartigen Ausgestaltung reicht es dann aus, dass quasi nur in eine Richtung und somit nur in eine Koordinatenachsenrichtung eine Zwischen-Verbindungsgerade erzeugt wird. Dies, da an dem Punkt des Wertepaares aus der Tiefe der vorderen Augenkammer des Patientenauges und der Länge des Patientenauges der Wert der horizontalen Tiefenlage allein aus dieser Geraden berechnet werden kann und gleich dem berechneten Wert ist, der sich durch eine in die zweite Koordinatenrichtung erzeugte weitere Zwischen-Verbindungsgeraden ergibt. Dies da sich aufgrund der eben ausgebildeten Teilfläche sich dies beiden Zwischen-Verbindungsgeraden genau an dem Punkt des vorherzusagenden horizontalen Tiefenlagenwerts schneiden.

Sind die Rand-Verbindungsgeraden so orientiert, dass sich zwischen ihnen eine Teilmasche mit unebener Teilfläche aufspannt, so kann durch oben erläuterte Vorgehensweise mit nur einer Zwischen-Verbindungsgeraden eine Ungenauigkeit in der Vorhersage auftreten. Da gerade durch die unebene Ausgestaltung des Vorhersagenetzes und auch der jeweiligen Teilmaschen zwei Zwischen-Verbindungsgeraden sich üblicherweise bezüglich des Werts der horizontalen Tiefenlage der Intraokularlinse im Patientenauge bei dem Wertepaar aus gemessener Tiefe der vorderen Augenkammer des Patientenauges und gemessener Länge des Patientenauges nicht schneiden, kann diesbezüglich im Weiteren ein weiteres Entscheidungsszenario berücksichtigt werden, um die präoperative Vorhersage zu verbessern.

Insbesondere kann dabei vorgesehen sein, dass an der Stelle des Wertepaars der Tiefe der vorderen Augenkammer und der Länge des Patientenauges die berechneten Werte der postoperativen Tiefenlage der Intraokularlinse im Patientenauge aus den beiden Zwischen-Verbindungsgeraden verglichen werden und abhängig von einer auftretenden Differenz der Werte der Wert der postoperativen Tiefenlage der Intraokularlinse im Patientenauge bestimmt wird. Dabei können unterschiedliche Vorgehensweisen vorgesehen sein. Die Bestimmung kann dabei einerseits von der Größe der Differenz abhängig sein. Zusätzlich oder anstatt dazu kann jedoch auch vorgesehen sein, dass bei Auftreten einer derartigen Differenz die weitere Bestimmung abhängig von dem Quadranten bzw. der Teilmasche im Vorhersagenetz erfolgt, dem das Wertepaar zugeordnet war bzw. von dem das Wertepaar umfasst war. Es kann dies abhängig von der Lage der Teilmasche in dem Vorhersagenetz und/oder abhängig von der Größe der Teilmasche im Vergleich zu anderen Teilmaschen und/oder im Vergleich zum Vorhersagenetz erfolgen.

Insbesondere ist vorgesehen, dass zur weiteren Bestimmung eine Mittelung des Differenzwerts durchgeführt wird, wobei insbesondere vorgesehen ist, dass der Differenzwert halbiert wird. Dieser so gemittelte Wert kann dann insbesondere dem kleineren der durch die Zwischen-Geraden berechneten Wert der postoperativ vorhergesagten horizontalen Tiefenlage hinzuaddiert werden.

Alternativ kann auch vorgesehen sein, dass dieser gemittelte Differenzwert dann dem größeren der beiden durch die Zwischen-Verbindungsgeraden berechneten Wert der horizontalen Tiefenlage abgezogen wird. Auch diese Addition oder Subtraktion des gemittelten Differenzwerts kann wiederum abhängig davon erfolgen, in welcher Teilmasche sich die Berechnung abspielt bzw. von welcher Teilmasche das gemessene Wertepaar des Patientenauges umfasst wird.

Es kann vorgesehen sein, dass die Anzahl der Haupt-Stützstellen und der Rand-Verbindungsgeraden so groß gewählt wird, dass das Vorhersagenetz eine zusammenhängende Vorhersagefläche bildet, die sich uneben im Raum erstreckt. Insbesondere kann ausgehend von dem Vorhersagenetz eine derartige Vorhersagefläche auch durch Interpolation abhängig von den Haupt-Stützstellen und den Rand-Verbindungsgeraden gebildet werden. Zusätzlich können auch Zwischen-Verbindungsgeraden für die Erzeugung der Vorhersagefläche berücksichtigt werden.

Insbesondere stellt der gemäß obiger Erläuterung ermittelte postoperative Tiefenlagenwert einen Vorhersagepunkt auf der Vorhersagefläche dar.

Es kann somit dann auch vorgesehen sein, dass im Fortgang der Vorhersage eine derartig große Anzahl an postoperativ bestimmten Tiefenlagenwerten vorliegt, dass aus diesen berechneten Tiefenlagenwerten die Vorhersagefläche gebildet wird, sodass dann im Nachgang für weitere Augen anhand der gemessenen Wertepaare für die Tiefe der vorderen Augenkammer und die Länge des Auges bereits aus dieser uneben gewölbten Vorhersagefläche der postoperative Tiefenlagenwert präoperativ vorhergesagt werden kann, ohne dass zusätzlich weitere Berechnungen mit Zwischen- und Verbindungsgeraden erfolgen müssen.

Vorzugsweise wird vorgesehen, dass für die Bestimmung des postoperativen Tiefenlagenwerts der Intraokularlinse im Patientenauge für eine vorgesehene Intraokularlinse mit einer gegenüber der vertikalen geneigten Haptik und/oder für einen spezifischen vorgesehenen Implantationsort der Intraokularlinse im Patientenauge ein erster Korrekturfaktor berücksichtigt wird. Die Berechnung des postoperativen Tiefenlagenwerts umfasst dann bei dem funktionellen Zusammenhang diesen ersten Korrekturfaktor.

Insbesondere wird ein Korrekturfaktor berücksichtigt, der abhängig von der Linsenform und/oder des Implantationsorts im Auge variabel bestimmt wird. Dadurch kann die Vorhersagepräzision nochmals erhöht werden. Insbesondere für ganz spezifische Kombinationen von erforderlichen Linsen zur Korrektur von entsprechenden Sehfehlern in Verbindung mit unterschiedlichsten Ausgestaltungen eines chirurgisch zu behandelnden Auges ermöglicht dann eine noch präzisere Vorhersage der postoperativen Lage, sodass die daran anschließende Modellierung auf Basis dieses präoperativ vorhergesagten Tiefenlagenwerts ebenfalls sehr präzise ermöglicht wird.

Vorzugsweise wird vorgesehen, dass der erste Korrekturfaktor durch folgenden funktionellen Zusammenhang bestimmt wird:

$$Fak_{korr} = -a + \left[ (Fak_Z \cdot b) - c \right],$$

wobei insbesondere a zwischen 1,3 und 1,43 liegt, b zwischen 0,45 und 0,65 und c zwischen 60 und 70, vorzugsweise zwischen 64 und 66 liegt.

Insbesondere gilt folgender Zusammenhang:

$$Fak_{korr} = -1,39329 + \left[ (Fak_Z \cdot 0,5663) - 65,60 \right]$$

Dabei ist $Fak_Z$ ein Zahlenwert, der von dem Linsentyp und/oder dem Ort, an dem die Linse im Auge implantiert werden soll, abhängig ist. Er kann auch eine Funktion von zumindest einem Linsenparameter sein.

Vorzugsweise ist darüber hinaus vorgesehen, dass die bereitgestellte Datenbasis bezüglich der Werte der Tiefe einer vorderen Augenkammer der anderen Augen kompatibel und einheitlich gestaltet wird. Da es diesbezüglich unterschiedliche Vorgehensweisen gibt, können diese Werte auf Basis unterschiedlicher Annahmen gemessen worden sein. Denn es ist in diesem Zusammenhang möglich, dass diese Tiefe der vorderen Augenkammer, bis zur Epithelschicht der Hornhaut oder bis zur Endothelschicht der Hornhaut gemessen wird. Da die Hornhaut eine durchschnittliche Dicke von 0,50 mm aufweist, sind diese erhaltenen Werte entsprechend anzugleichen, sodass die in der Datenbasis bereitgestellten Werte der anderen Humanaugen auf gleichen Annahmen bezüglich der Messlänge basieren.

Zur weiteren Vorgehensweise der Berechnung der postoperativen horizontalen Tiefenlage einer Intraokularlinse im Patientenauge wird dann auf Basis der gemessenen Werte der Tiefe der vorderen Augenkammer und der Länge des Patientenauges der Quadrant bzw. die Teilmasche des Vorhersagenetzes ausgewählt, in dem diese gemessenen Werte des Patientenauges bezüglich dieser beiden Parameter umfasst ist.

In einem weiteren Schritt wird dann ausgehend von den bestimmten Rand-Verbindungsgeraden dieser spezifischen Teilmasche, die gemessenen Werte des Patientenauges umfasst, die postoperative horizontale Tiefenlage der Intraokularlinse im Patientenauge berechnet und kann somit dann präoperativ vorhergesagt werden.

Vorzugsweise wird das Vorhersagenetz und/oder die bestimmte und präoperativ vorhergesagte postoperative horizontale Tiefenlage der Intraokularlinse auf einer Anzeigeeinheit einer Vorrichtung angezeigt. Insbesondere wird die bestimmte und präoperativ vorhergesagte postoperative horizontale Tiefenlage der Intraokularlinse auf einer Anzeigeeinheit einer Vorrichtung als Wert und/oder in einem als Bild dargestellten Auge angezeigt werden. Dazu kann ein symbolisches Schnittbild oder aber auch ein Echtbild vorgesehen sein. Die bestimmte Tiefenlage kann auch als eine Overlay-Information bei einem Bild auf der Anzeigeeinheit angezeigt werden.

Diese Zusammenhänge sind dabei für eine Intraokularlinse gültig, deren Haptik nicht gegenüber der Vertikalen geneigt ist und die in dem Kapselsack des Auges implantiert werden soll. Soll eine Intraokularlinse mit geneigter Haptik im ersten Auge implantiert werden und/oder die Implantation nicht im Kapselsack, sondern in der vorderen Augenkammer oder der Zilliarfurche implantiert werden, so ist die Berechnung unter Berücksichtigung des bereits oben angesprochenen Korrekturfaktors durchzuführen.

Insbesondere wird der Z-Faktor $Fak_Z$, von dem der erste Korrekturfaktor abhängig ist, mit Werten zwischen 110 und 125 festgelegt.

Insbesondere ist dabei vorgesehen, dass dieser Z-Faktor $Fak_Z$ wertmäßig abhängig vom Typ der Intraokularlinse variiert. Beispielsweise beträgt der Z-Faktor 115,15 für eine in der vorderen Augenkammer implantierte Intraokularlinse. Er beträgt 115,60 für eine irisfixierte Intraokularlinse. Er beträgt insbesondere 116,05 für eine sulkusfixierte Intraokularlinse, welche plankonvex ist. Er beträgt insbesondere 116,90 für eine sulkusfixierte Intraokularlinse, welche bikonvex geformt ist. Er beträgt insbesondere 117,65 für eine im Kapselsack fixierte Intraokularlinse, welche plankonvex geformt ist. Er beträgt insbesondere 118,30 für eine im Kapselsack fixierte Intraokularlinse, welche bikonvex geformt ist.

Es kann auch vorgesehen sein, dass ein einmal erzeugtes Vorhersagenetz abhängig von nachträglich erhaltenen Informationen über die tatsächliche positionelle Tiefenlage der Intraokularlinse im Patientenauge korrigiert wird und insbesondere entsprechende Haupt-Stützstellen verändert werden. Insbesondere können dann auch die Vorhersagepunkte auf der Vorhersagefläche entsprechend geändert werden, so dass sich die unebene Formgebung anhand von nachträglich gemessenen tatsächlichen Werten des Auges anpassen lässt.

In einer vorteilhaften Ausführung kann vorgesehen sein, dass eine Sortierung unter Berücksichtigung von Radien der Hornhäute von Augen erfolgt. Insbesondere können dann auch mehrere Matrizen als Vorhersagenetze erstellt werden. Insbesondere bedeutet dies, dass somit ein Vorhersagenetz für einen ganz spezifischen Radius einer Hornhaut erzeugt wird, wie dies oben anhand der Abläufe dargelegt wurde. Insbesondere kann dann für jeden Radius ein eigenes Vorhersagenetz erstellt werden.

Darüber hinaus können auch weitere Parameter, wie beispielsweise die Dicke der natürlichen Linse eines Auges und/oder das Alter des Patienten oder die ethnische Herkunft berücksichtigt werden. Als weitere biometrische Daten kann neben der Hornhautkrümmung auch die Begrenzung der Augenlider eines Auges, das so genannte WTW (White to White)-Verhältnis berücksichtigt werden.

Insbesondere wird für die Modellierung eines Auges bei der Berechnung der Brechkraft für die Intraokularlinse das Gullstrandt-Modell zugrunde gelegt. Es sei explizit darauf hingewiesen, dass die Erfindung nicht auf ein spezifisches Augenmodell fixiert ist, sondern unabhängig von einem spezifischen Augenmodell eine präoperative Vorhersage möglich ist. Es können als weitere Augenmodelle auch beispielsweise das Liou-Brennan-Modell oder das Holladay-Modell zugrunde gelegt werden. Dies sind nur beispielhafte Nennungen für Modelle, wobei die Aufzählung nicht abschließend zu verstehen ist.

Die Erfindung betrifft auch ein Verfahren zum Modellieren einer Augenlinse, insbesondere einer Intraokularlinse, bei welchem die Form und/oder Brechkraft der Augenlinse bestimmt werden und dazu eine postoperative horizontale Tiefenlage der Augenlinse in dem Patientenauge, in dem die Augenlinse implantiert werden soll, berücksichtigt wird, wobei diese Tiefenlage durch ein oben erläutertes Verfahren gemäß der Erfindung oder einer vorteilhaften Ausführung davon bestimmt bzw. vorhergesagt wird. Die zu implantierende Augenlinse wir dann anhand der Modellierung präoperativ ausgewählt.

Die Erfindung betrifft auch eine Vorrichtung bzw. ein System zur präoperativen Vorhersage einer postoperativen horizontalen Tiefenlage einer Intraokularlinse in einem Patientenauge, welches zum Durchführen des erfindungsgemäßen Verfahrens oder einer vorteilhaften Ausgestaltung davon ausgebildet ist. Das System beziehungsweise die Vorrichtung umfasst dazu zumindest eine Prozessoreinheit und ein computerlesbares Speichermedium, welches zur Kommunikation mit dem Prozessor ausgebildet ist.

Insbesondere ist vorgesehen, dass das System beziehungsweise die Vorrichtung einen Speicher aufweist, in dem eine Vielzahl von Werten betreffend die Tiefen der vorderen Augenkammer von anderen Humanaugen, Werte der horizontalen Längen dieser anderen Humanaugen und Werte von postoperativen Tiefenlagen von Intraokularlinsen in diesen anderen Humanaugen gespeichert sind. Der Speicher kann Bestandteil des Systems und der Vorrichtung sein, kann jedoch auch ein externer Speicher sein, der mit dem System beziehungsweise der Vorrichtung koppelbar ist.

Das System umfasst darüber hinaus eine Mehrzahl von Instruktionssequenzen, die zur Verarbeitung mit dem Prozessor vorgesehen sind. Diese Instruktionen sind so aufgebaut, dass der Prozessor dahingehend gesteuert wird, dass er aus Werten spezifischer Wertetripel dieser oben genannten Parameter Haupt-Stützstellen auswählt und bildet. Vorzugsweise werden die Haupt-Stützstellen jedoch nutzerdefiniert vorgegeben. Darüber hinaus sind die Instruktionen dahingehend ausgelegt, dass diese Haupt-Stützstellen von dem Prozessor in ein dreidimensionales Koordinatensystem eingetragen werden, wobei dieses Koordinatensystem an den drei Koordinatenachsen durch die Parameter betreffend die Tiefe einer vorderen Augenkammer, die Länge eines Auges und die postoperative horizontale Tiefenlage der Intraokularlinse aufgespannt ist.

Darüber hinaus sind diese Instruktionssequenzen so ausgebildet, dass der Prozessor dahingehend gesteuert ist, dass Haupt-Stützstellen durch Rand-Verbindungsgeraden verbunden werden. Diese Rand-Verbindungsgeraden werden abhängig von den Haupt-Stützstellen durch den Prozessor und die hinterlegten Instruktionssequenzen berechnet.

Durch die Haupt-Stützstellen und die Rand-Verbindungsgeraden ist dann ein im dreidimensionalen Koordinatensystem uneben geformtes und insbesondere simulativ gebildetes Vorhersagenetz ausgebildet, welches zur Vorhersage der postoperativen horizontalen Tiefenlage der Intraokularlinse im Patientenauge erzeugt ist.

Insbesondere ist das System mit weiteren Instruktionssequenzen ausgebildet, die dann ausgehend von eingegebenen gemessenen Werten der Tiefe der vorderen Augenkammer und der Länge des Patientenauges abhängig von dem Vorhersagenetz und insbesondere von einer oder mehreren Rand-Verbindungsgeraden und Zwischenverbindungsgeraden eine postoperative horizontale Tiefenlage der Intraokularlinse im Patientenauge bestimmt, insbesondere berechnet.

Dafür sind weitere Instruktionssequenzen vorgesehen, mittels denen der Prozessor dahingehend steuerbar ist, dass er abhängig von diesen eingegebenen und gemessenen Werten des Patientenauges Zwischen-Verbindungsgeraden auf Basis und abhängig von den Rand-Verbindungsgeraden einer spezifischen Teilmasche beziehungsweise eines spezifischen Quadranten des Vorhersagenetzes berechnet und abhängig davon dann den präoperativ vorherzusagenden postoperativen horizontalen Tiefenlagenwert der Intraokularlinse im Patientenauge bestimmt, insbesondere berechnet.

Vorzugsweise sind weitere Instruktionssequenzen vorgesehen, die zur Abarbeitung der Verfahrensschritte des erfindungsgemäßen Verfahrens oder in vorteilhafter Ausgestaltung davon ausgebildet sind.

Vorzugsweise umfasst das System auch abgelegte Tabellen über Verbindungsgeraden und spezifische Aufteilungen von Quadranten beziehungsweise Teilmaschen des Vorhersagenetzes.

Das System ist insbesondere auch dazu ausgebildet, dass es abhängig von der präoperativ vorhergesagten postoperativen horizontalen Tiefenlage der Intraokularlinse in einem zu betrachtenden Patientenauge, bei dem die natürliche Linse entfernt und durch eine Intraokularlinse ersetzt wird, eine Bestimmung, insbesondere Berechnung, der Brechkraft und/oder der Linsenform durchführen kann.

Insbesondere abhängig von dieser modellierten Berechnung der Intraokularlinse wird diese dann hergestellt und im Weiteren dann in das Patientenauge implantiert.

Vorzugsweise ist vorgesehen, dass die oben angegebenen Formeln und Zusammenhänge in einem Speicher des Systems abgelegt sind.

Besonders bevorzugt ist vorgesehen, dass der Z-Faktor $Fak_z$ nicht als konkreter statischer Wert vorgegeben ist, sondern dass dieser eine Funktion eines Parameters der Intraokularlinse, insbesondere des Parameters der Brechkraft, ist. Vorzugsweise ist hier eine lineare Funktion zugrunde gelegt.

Insbesondere ist vorgesehen, dass bezüglich des Parameters einer postoperativen horizontalen Tiefenlage einer Intraokularlinse der horizontale Weg, beziehungsweise die horizontale Strecke zwischen der Außenseite der Hornhaut und dem Äquator beziehungsweise der vertikalen Mittenebene der Intraokularlinse bezeichnet ist. Prinzipiell kann jedoch auch eine anderweitige Definition zugrunde gelegt werden, wobei dann im Hinblick auf die Kompatibilität und die Vergleichbarkeit der Werte entsprechende Korrekturfaktoren berücksichtigt werden müssen. Dies betrifft insbesondere Ausgestaltungen, bei denen dieser Parameter nicht von der Vorderseite der Hornhaut sondern von der Rückseite der Hornhaut ab betrachtet wird. Ebenso ist dies entsprechend zu korrigieren, wenn dieser Parameter der horizontalen Tiefenlage sich dahingehend bemisst, dass er nicht bis zum Äquator der Linse sondern nur bis zur Vorderseite der Linse charakterisiert ist.

Darüber hinaus ist zu erwähnen, dass auch der Parameter der Tiefe einer vorderen Augenkammer definitionsgemäß unterschiedlich festgelegt werden kann. Theoretisch betrachtet ist die Tiefe der vorderen Augenkammer, von der Rückfläche der Hornhaut des Auges bis zur Vorderfläche der Iris definiert. Zwischen diesen beiden Grenzflächen befindet sich die vordere Augenkammer. Zwischen der Rückfläche der Iris und dem Glaskörper des Auges befindet sich die hintere Augenkammer. Messtechnisch kann die vordere Augenkammer mittels Ultraschall sowie mit Licht, beispielsweise Laserinterferometrie, vermessen werden. Beide Verfahren unterscheiden sich geringfügig. Zum einen in der physikalischen Art der Anwendung sowie im Messergebnis. Beiden Verfahren gleich ist die Bemessung der Strecke von einer Grenzfläche zur nächsten Grenzfläche. Eine Grenzfläche wird umso genauer definiert, je größer die Unterschiede zwischen den beiden Medien hinsichtlich Licht- und Schallgeschwindigkeit sind. Es kann also von der vorderen Hornhautfläche oder auch von der hinteren Hornhautfläche und somit von der Vorderseite beziehungsweise der Rückseite der Hornhaut gemessen werden. Der Unterschied der Grenzmedien ist jedoch an der Vorderseite der Hornhaut am größten. Demnach ist das Auffinden der Grenzfläche am exaktesten an der Vorderseite der Hornhaut.

Eine präoperativ gemessene Tiefe der vorderen Augenkammer kann somit definitionsgemäß dahingehend wegmäßig festgesetzt werden, dass sie von der Vorderseite der Hornhaut bis zur Vorderseite der natürlichen Augenlinse bemessen wird. Dies ist beispielsweise bei dem von der Anmelderin bekannten System IOL-Master vorgesehen.

Auch hier kann jedoch eine anderweitige Definition zugrunde gelegt werden und die Messung von der Rückfläche der Hornhaut bis zur Vorderseite der natürlichen Linse erfolgen. Wird dann noch die Dicke der Hornhaut gemessen, kann wiederum ein kompatibler Wertevergleich zwischen den beiden möglichen Definitionen hergestellt werden. Erfahrungsgemäß kann als sehr guter Mittelwert eine Hornhautdicke von 0,5 mm angenommen werden.

Analog kann die postoperativ gemessene Tiefe der vorderen Augenkammer von der Vorderseite der Hornhaut bis zur Vorderseite der künstlichen Augenlinse und somit der Intra-okularlinse definiert werden. Auch hier kann jedoch auch definitionsgemäß von der Rückseite der Hornhaut bis zur Vorderseite der künstlichen Augenlinse gemessen werden.

Bezüglich der bereits oben angegebenen Erläuterungen ist für den Parameter der postoperativen horizontalen Tiefenlage einer Intraokularlinse in einem ersten Auge, der präoperativ vorhergesagt werden soll, eine Wegstrecke auf der horizontalen Hauptlinie des Auges vorgesehen, die sich von der Vorderseite der Hornhaut bis zur vertikalen Mittenebene und somit zum Äquator der Intraokularlinse erstreckt. Dies ist dann insbesondere auch der Äquator und somit die vertikale Mittenebene des Kapselsackes. Wird eine Intraokularlinse vorgesehen, die eine nicht gewinkelte Haptik aufweist, so fallen die Haptikenden mit dem Äquator der Intraokularlinse zusammen. Insbesondere dann, wenn die Intraokularlinse auch noch eine bisymmetrisch verteilte Brechkraft aufweist, entspricht der durch das Verfahren vorhergesagte postoperative horizontale Tiefenlagenwert der Intraokularlinse im ersten Auge der Entfernung der vorderen Hornhautfläche zur Mitte der Intraokularlinse.

Wie bereits oben ebenfalls erläutert, ist ein erster Korrekturfaktor vorzusehen, wenn die Haptik der Intraokularlinse angewinkelt ist und/oder die Intraokularlinse nicht im Kapselsack implantiert werden soll. Insbesondere ist dann auch ein Korrekturfaktor zu verwenden, wenn die Brechkraft der vorgesehenen Intraokularlinse auf beiden Krümmungsflächen asymmetrisch ist. In derartigen Ausgestaltungen ist die Position des vertikalen Äquators der Intraokularlinse anzupassen, was mit dem bereits oben erläuterten Z-Faktor $Fak_z$ erfolgt.

Wird insbesondere bei der Vorgehensweise zur präoperativen Vorhersage der postoperativen horizontalen Tiefenlage einer Intraokularlinse im Auge davon ausgegangen, dass der vertikale Äquator der natürlichen Linse sich immer an einer bestimmten Stelle befindet, und diese Position unabhängig vom zu implantierenden Typ oder Modell einer Intraokularlinse ist, kann diese Stelle auch im voraus berechnet werden, was insbesondere anhand von Messwerten der Augenlänge, der Vorderkammertiefe, der Krümmung der Hornhaut, der Dicke der natürlichen Linse und gegebenenfalls noch weiteren Parametern erfolgen kann.

Für die grundsätzliche Ausgangslage zur Vorhersage dieser horizontalen Tiefenlage wird davon ausgegangen, dass die Linse eine nicht gewinkelte Haptik aufweist und im Kapselsack implantiert wird, sodass dann der vertikale Äquator bzw. die vertikale Äquatorebene der Intraokularlinse quasi mit der Hauptebene zusammenfällt, so befindet sich die Hauptebene der Intraokularlinse an der gleichen Position wie der Äquator des Kapselsacks.

Entsprechende Korrekturen bei der zu berechnenden präoperativen Vorhersage im Hinblick auf angewinkelte Haptik und/oder Implantationen an einer anderen Stelle als dem Kapselsack und/oder asymmetrisch verteilte Brechkraft der Vorder- und der Rückseite der Intraokularlinse, lässt sich bezüglich der präoperativen Vorhersage dieser Tiefenlage durch die genannten Korrekturfaktoren abbilden.

Die Erfindung betrifft darüber hinaus auch ein Computerprogrammprodukt, auf dem Instruktionssequenzen zum Durchführen des erfindungsgemäßen Verfahrens oder einer vorteilhaften Ausgestaltung davon abgelegt, insbesondere gespeichert sind.

**Kurze Beschreibung der Zeichnung(en)**

Ein Ausführungsbeispiel der Erfindung wird nachfolgend anhand schematischer Zeichnungen näher erläutert. Es zeigen:

Fig. 1    einen Vertikalschnitt durch ein Auge, indem spezifische Parameter, die zur präoperativen Vorhersage benötigt werden, dargestellt sind;

Fig. 2    eine vergrößerte Vertikalschnittdarstellung eines Auges mit einer implantierten Intraokularlinse und der Darstellung des Parameters der postoperativen horizontalen Tiefenlage postopeI-OLE der Intraokularlinse mit ungewinkelten Haptiken im Auge;

Fig. 3    eine schematische Darstellung eines dreidimensionalen Koordinatensystems mit einem beispielhaften Vorhersagenetz;

Fig. 4    eine Projektionsdarstellung des Vorhersagenetzes gemäß Fig. 3 in eine Ebene, die durch die Koordinatenachsen aufgespannt ist, auf denen die Parameter der Tiefe der vorderen Augenkammer und der Länge eines Auges aufgetragen sind;

Fig. 5    eine beispielhafte Darstellung eines uneben im Raum geformten Vorhersagenetzes mit der Einzeichnung von Zwischen-Verbindungsgeraden;

Fig. 6    ein weiteres Ausführungsbeispiel eines dreidimensionalen Koordinatensystems mit einem uneben geformten Vorhersagenetz;

Fig. 7    eine vergrößerte Vertikalschnittdarstellung eines Auges mit einer implantierten Intraokularlinse und der Darstellung des Parameters der postoperativen horizontalen Tiefenlage postopeI-OLE* der Intraokularlinse mit gewinkelten Haptiken im Auge;

**Bevorzugte Ausführung der Erfindung**

In den Figuren werden gleiche oder funktionsgleiche Elemente mit den gleichen Bezugszeichen versehen.

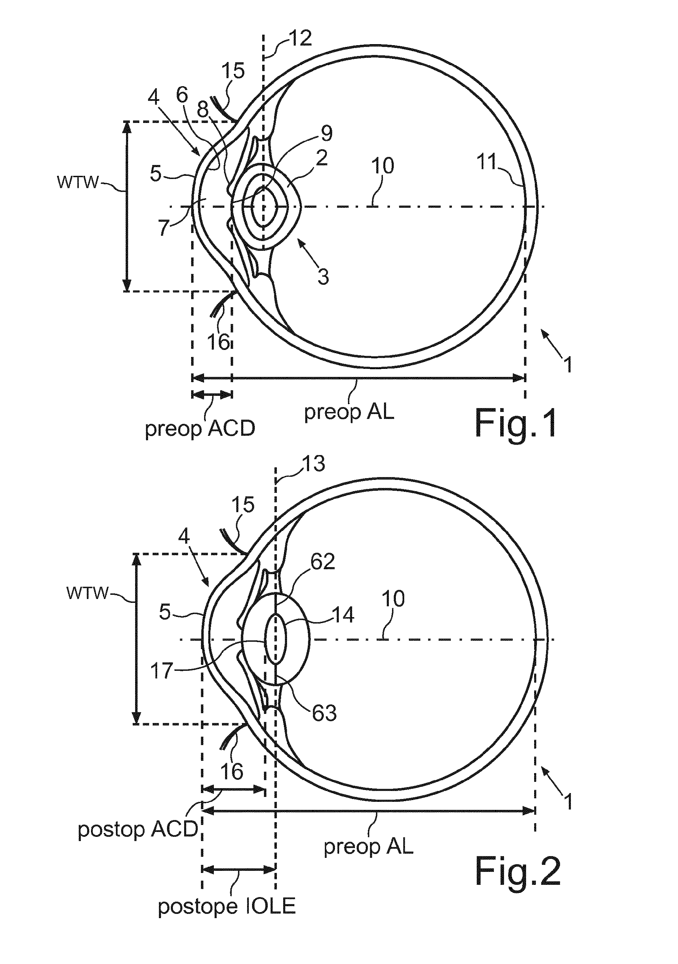

In Fig. 1 ist in einer schematischen Vertikalschnittdarstellung ein erstes Auge bzw. Patientenauge 1 gezeigt, welches einen zu korrigierenden Sehfehler aufweist. In einem chirurgischen Eingriff ist vorgesehen, eine natürliche Linse 2, die in einem Kapselsack 3 angeordnet ist, zu entfernen. Es soll dann eine künstliche Augenlinse in Form einer Intraokularlinse eingesetzt beziehungsweise implantiert werden. Das Auge 1 weist eine Hornhaut 4 auf, die eine Vorderseite 5 und eine Rückseite 6 hat. Anschließend an die Hornhaut 4 ist eine vordere Augenkammer 7 ausgebildet.

Darüber hinaus ist die Iris 8 des Auges 1 eingezeichnet.

Die natürliche Linse 2 weist ebenfalls eine Vorderseite 9 auf, die somit der Hornhaut 4 zugewandt ist.

Im gezeigten Ausführungsbeispiel ist vorgesehen, dass entlang einer horizontalen Mittenlinie 10 und somit der optischen Hauptachse 10 des Auges 1 der Parameter der horizontalen Tiefe der vorderen Augenkammer 7 bemessen ist. Dieser Parameter wird nachfolgend als preopACD bezeichnet.

Er bemisst sich im Ausführungsbeispiel auf der optischen Achse 10 des Auges 1 von der Vorderseite 5 der Hornhaut 4 bis zur Vorderseite 9 der natürlichen Linse 2. Er wird durch bereits oben erwähnte Verfahren gemessen.

Darüber hinaus wird auch ein Wert des Parameters der horizontalen Länge des Auges 1 bestimmt. Dieser Parameter wird als preopAL bezeichnet und bemisst sich ebenfalls entlang der optischen Achse 10. Er erstreckt sich im Ausführungsbeispiel von der Vorderseite 5 der Hornhaut 4 bis zur Netzhaut 11 des Auges 1. Auch dieser Wert wird für das Patientenauge 1 präoperativ gemessen.

Darüber hinaus ist in der Darstellung gemäß Fig. 1 eine vertikale Mittenebene, auch als vertikaler Äquator 12 (Äquatorebene) bezeichnet, des Kapselsacks 3 dargestellt.

Um nun den Sehfehler dieses Patientenauges 1 bestmöglich zu korrigieren ist es erforderlich, die Position und die Ausgestaltung der zu implantierenden Intraokularlinse präoperativ bestmöglich vorhersagen und modellieren zu können.

Zur präoperativen Vorhersage einer postoperativen horizontalen Tiefenlage einer zu implantierenden Intraokularlinse in diesem Auge 1 wird nachfolgend die entsprechende Vorgehensweise zur Vorhersage näher erläutert.

Dazu wird zunächst eine Vorrichtung beziehungsweise ein System bereitgestellt, welches eine Recheneinheit mit zumindest einem Prozessor und einer Speichereinheit aufweist. Mittels einer Eingabeeinheit können die gemessenen Werte der Parameter preopACD und preopAL des Auges 1 eingegeben werden.

In der Vorrichtung ist eine Mehrzahl von Instruktionssequenzen abgelegt, die beispielsweise auf einem Computerprogrammprodukt in Form eines Speichermediums abgelegt sein können. In der Vorrichtung ist eine Kommunikation zwischen dem Prozessor und dem Speichermedium gegeben, sodass entsprechend steuerbar ist, welche Instruktionssequenzen der Prozessor auswählen und abarbeiten soll und auf welcher Basis der eingegebenen und abgelegten Werte eine Berechnung zur präopertiven Vorhersage einer postopertiven horizontalen Tiefenlage einer Intraokularlinse in dem Auge 1 erfolgen soll.

In der Vorrichtung sind in einem Datenspeicher eine Vielzahl von Werten abgelegt, die Werte von dem Parameter preopACD und somit der Tiefe der vorderen Augenkammern von anderen Humanaugen und Werte des Parameters preopAL und somit von horizontalen Längen dieser anderen Humanaugen umfassen. Darüber hinaus sind in diesem Datenspeicher auch postoperativ gemessene Werte eines dritten Parameters, nämlich der postoperativen Tiefenlage von Intraokularlinsen in diesen anderen Humanaugen abgespeichert. Insbesondere sind jedoch geschätzte Werte der postoperativen horizontalen Tiefenlage von Intraokularlinsen bereitgestellt. Die Werte der anderen Humanaugen, die als Datenbasis zur weiteren Vorgehensweise bei der präoperativen Vorhersage der postoperativen horizontalen Tiefenlage eine Intraokularlinse im Patientenauge 1 herangezogen werden, werden somit gesammelt und abgespeichert und insbesondere auch stetig aktualisiert.

Es kann auch vorgesehen sein, dass Werte, insbesondere alle, geschätzt und/oder berechnet sind.

Im Hinblick auf den Parameter der horizontalen Tiefenlage einer Intraokularlinse, der gemäß der Darstellung in Fig. 2 mit postopeIOLE bezeichnet wird, bemisst sich dieser im Ausführungsbeispiel von der Vorderseite 5 der Hornhaut 4 bis zu einer vertikalen Mittenebene 13, auch als vertikaler Äquator oder Hauptebene bezeichnet, der Intraokularlinse 14.

Gemäß der Darstellung in Fig. 2 ist die modellhafte Darstellung des Auges 1 gezeigt, sodass dies auch insbesondere simulativ modelliert gezeigt ist.

Insbesondere kann bei der präoperativen Vorhersagebestimmung des Tiefenlagenwerts und somit des Werts des Parameters postopeIOLE eine Berücksichtigung von weiteren biometrischen Parametern des Auges 1 erfolgen. Als biometrische Parameter können hierbei die Krümmung der Hornhaut 4, die Dicke der natürlichen Linse 2 entlang der optischen Achse 10 gemessen und das WTW-Verhältnis berücksichtigt werden. Das WTW-Verhältnis bemisst sich dabei in vertikaler Richtung zwischen den Anlagepunkten der Augenlider 15 und 16 an dem Auge 1.

Zur weiteren Erläuterung der Vorgehensweise zur präoperativen Vorhersage des Wertes für den Parameter postopeIOLE für das Ausführungsbeispiel in Fig. 2 wird dann ausgehend von der zur Verfügung gestellten Datenbasis von einer Vielzahl von anderen Humanaugen eine Auswahl von Haupt-Stützstellen durchgeführt. Dabei werden auf Basis der zur Verfügung stehenden Werte für die drei genannten Parameter preopACD und preopAL sowie für die anderen Augen postoperativ gemessene Werte von horizontalen Tiefenlagen einer Intraokularlinse Wertetripel gebildet.

Insbesondere werden die Haupt-Stützstellen auf Basis von geschätzten Werten der Parameter und/oder von gemessenen Werten dieser Parameter an anderen Humanaugen als dem Patientenauge gebildet, insbesondere nutzerausgewählt gebildet. Wann geschätzte und/oder wann gemessene Werte berücksichtigt werden hängt von der jeweils zu generierenden Haupt-Stützstelle ab, insbesondere deren Lage von den anderen Haupt-Stützstellen ab. Insbesondere werden die Werte der Vorderkammertiefe und die Werte der Augenlänge der anderen Augen nur gemessen.

Wie auch in der Darstellung gemäß Fig. 2 beispielhaft eingezeichnet, bemisst sich dieser postoperativ gemessene Wert der Zwischenlage, der als Parameter durch postopACD bezeichnet ist, zwischen der Vorderseite 5 der Hornhaut 4 und der Vorderseite 17 der Intraokularlinse 14.

Es ist zu erwähnen, dass die Darstellung in Fig. 2 eine bereits implantierte IOL zeigt, um die einzelnen Parameter darstellen und erläutern zu können. Natürlich ist die IOL im Auge 1 noch nicht implantiert, wenn das erfindungsgemäße Verfahren zur präoperativen Vorhersage der postoperativen horizontalen Tiefenlage durchgeführt wird.

Im Hinblick auf die bereitgestellte und abgespeicherte Datenbasis dieser Wertetripel von den anderen Humanaugen ist zunächst auch noch abzugleichen, ob diese erhaltenen Werte auch kompatibel sind und auf gleicher Annahme der Weglängendefinitionen begründen. Wie bereits oben angesprochen, können diesbezüglich unterschiedliche Definitionen zugrunde liegen, und die Kompatibilität ist dahingehend herzustellen, dass gegebenenfalls auch noch die Dicke der Hornhaut 4, insbesondere entlang der optischen Achse 10 berücksichtigt wird.

Die Datenbasis ist somit dann dahingehend bereitzustellen, dass die Werte, insbesondere alle Werte auf der gleichen Definitionsgrundlage für die Parameter preopACD, preopAL und postopeIOLE bereitstehen.

Wie bereits angesprochen, wird dann aus spezifischen Wertetripeln eine Mehrzahl von Hauptstützstellen gebildet, die dann in ein dreidimensionales Koordinatensystem eingezeichnet werden.

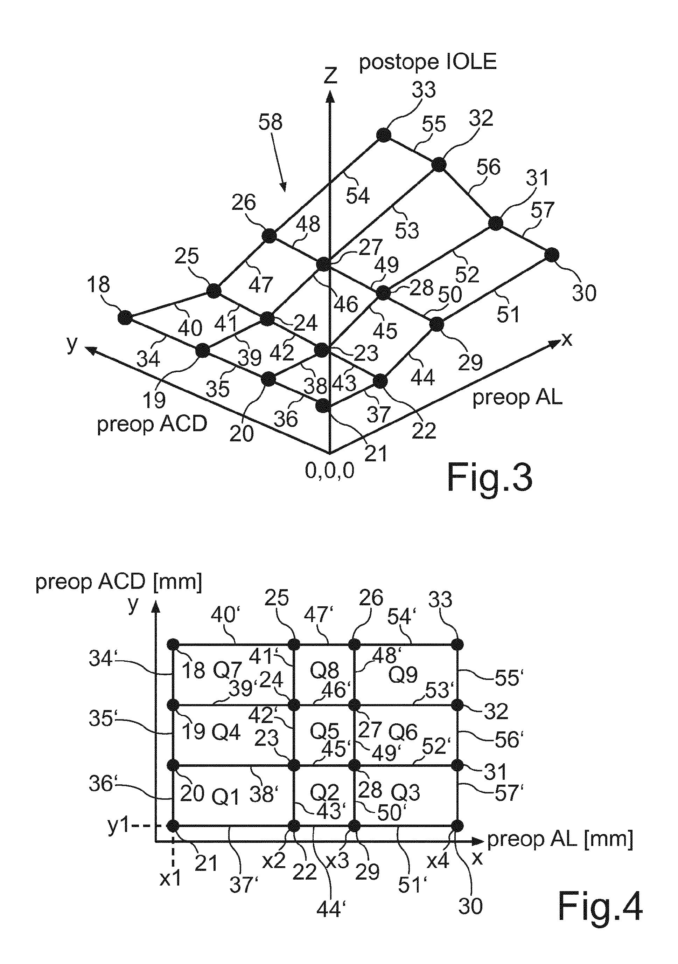

Dies ist in Fig. 3 dargestellt, wobei dabei auf der x-Achse der Parameter preopAL, auf der y-Achse der Parameter preopACD und auf der z-Achse der Parameter postopeIOLE aufgetragen ist. Die Koordinatenachsen stehen senkrecht aufeinander.

Aus den ausgewählten Wertetripeln werden dann die bereits angesprochenen Stützstellen in dieses dreidimensionale Koordinatensystem eingezeichnet. Im Ausführungsbeispiel ist vorgesehen, dass 16 Haupt-Stützstellen 18 bis 33 eingezeichnet werden.

Diese Haupt-Stützstellen 18 bis 33 sind im Ausführungsbeispiel so gewählt, dass sie jeweils Eckpunkte von rechteckigen Teilflächen darstellen, wenn diese in die x-y-Ebene projiziert werden. Dies ist in der Darstellung gemäß Fig. 4 gezeigt.

Die Haupt-Stützstellen 18 bis 33 werden dann durch Rand-Verbindungsgeraden verbunden. Diese Rand-Verbindungsgeraden werden berechnet, wobei dabei jeweils die benachbarten Haupt-Stützstellen berücksichtigt werden, so dass aus zwei benachbarten Haupt-Stützstellen eine Rand-Verbindungsgerade aufgrund einer linearen Gleichung berechnet wird. So werden Rand-Verbindungsgeraden 34 bis 57 gebildet. Dadurch entsteht das in Fig. 3 eingezeichnete im dreidimensionalen Raum uneben geformte Vorhersagenetz 58.

Aus der nachfolgenden Tabelle 1 sind beispielhafte Werte für die Haupt-Stützstellen 18 bis 33 zu entnehmen. Ebenso sind dort die Geradengleichungen für die Rand-Verbindungsgeraden in eine Koordinatenrichtung eingetragen.

Tabelle 1

| preop ACD | preop AL | 15,00 | | 22,50 | | 22,50 | | 25,00 | | 25,00 | | 40,00 |
|---|---|---|---|---|---|---|---|---|---|---|---|---|
| | Z | 2,250 | | 3, 100 | | 3,100 | | 3,500 | | 3,500 | | 4,50 |
| 1,50 | 2,250 | $Z^{x1}_{RV\,36}=0,9842857\cdot ACD+0,8035714$ | | | | $Z^{x2}_{RV\,43}=0,7142857\cdot ACD+2,0285714$ | | | | $Z^{x3}_{RV\,50}=0,75\cdot ACD+2,375$ | | |
| 2,90 | 3,600 | $Z^{x2}_{RV\,43}=0,7142857\cdot ACD+2,0285714$ | | | | $Z^{x3}_{RV\,50}=0,75\cdot ACD+2,375$ | | | | $Z^{x4}_{RV\,57}=0,2857143\cdot ACD+4,0714288$ | | |
| 2,90 | 3,600 | $Z^{x1}_{RV\,35}=0,5\cdot ACD+2,15$ | | | | $Z^{x2}_{RV\,42}=0,49988\cdot ACD+2,85348$ | | | | $Z^{x3}_{RV\,49}=0,0\cdot ACD+4,55$ | | |
| 3,40 | 3,850 | $Z^{x2}_{RV\,42}=0,49988\cdot ACD+2,650348$ | | | | $Z^{x3}_{RV\,49}=0,0\cdot ACD+4,55$ | | | | $Z^{x4}_{RV\,56}=0,7\cdot ACD+2,87$ | | |
| 3,40 | 3,850 | $Z^{x1}_{RV\,34}=0,8269231\cdot ACD+1,0384615$ | | | | $Z^{x2}_{RV\,41}=0,6346385\cdot ACD+2,1921692$ | | | | $Z^{x3}_{RV\,48}=0,5576923\cdot ACD+2,6538462$ | | |
| 6,00 | 6,000 | $Z^{x2}_{RV\,41}=0,6346385\cdot ACD+2,1921692$ | | | | $Z^{x3}_{RV\,48}=0,5576923\cdot ACD+2,6538462$ | | | | $Z^{x4}_{RV\,55}=0,7884615\cdot ACD+2,5692308$ | | |

Dabei bezeichnet Z die Funktion der Gleichung für die Rand-Verbindungsgerade (RV) an der Stelle xi. Z ist dabei eine Funktion von y, wobei y der Parameter preopACD ist. Der Übersichtlichkeit dienend ist Z ohne diese Variable angegeben und in der Gleichung lediglich ACD anstelle von preopACD geschrieben. Beispielhaft sind lediglich die Gleichungen für Geraden angegeben, welche sich in Fig. 4 parallel zur y-Achse erstrecken. Ebenso könnten auch die Gleichungen für die Geraden, die sich in Fig. 4 parallel zur x-Achse erstrecken angegeben werden.

Wie zu erkennen ist, wird das Vorhersagenetz 58 aus einer Mehrzahl von Teilflächen bzw. Teilnetzen aufgespannt, wobei ein Teilnetz aus zumindest einer Teilmasche aus jeweils durch vier benachbart zueinander angeordnete Haupt-Stützstellen 18 bis 33 und den jeweils diese vier Haupt-Stützstellen 18 bis 33 miteinander verbindenden Rand-Verbindungsgeraden 34 bis 57 begrenzt ist. Wie zu erkennen ist, ist das Vorhersagenetz 58 aus der Mehrzahl von direkt miteinander zusammenhängenden und an einander angrenzenden Teilflächen bzw. Teilnetzen aufgespannt. Werden die Teilnetze flächig betrachtet, so weisen sie unterschiedliche Steigungen und Krümmungen in dem dreidimensionalen Koordinatensystem auf.

Zum Ausführungsbeispiel sind zumindest einige Flächen von Teilmaschen uneben im Raum ausgebildet, was sich durch die Lage der zugehörigen Haupt-Stützstellen ergibt.

Wie aus der Darstellung in Fig. 4 zu erkennen ist, werden die Haupt-Stützstellen 18 bis 33 so ausgewählt, dass sie bei einer Projektion in die x-y-Ebene ein Rechteck aufspannen. Dazu ist vorgesehen, dass eine Mehrzahl von Stützstellen, im Ausführungsbeispiel die Stützstellen 18 bis 21, unterschiedliche preopACD-Werte jedoch einen gleichen preopAL-Wert aufweisen. Entsprechendes gilt für die Haupt-Stützstellen 22 bis 25. Diese Haupt-Stützstellen 22 bis 25 sind an einem unterschiedlichen preopAL-Wert ausgewählt, weisen jedoch die gleichen preopACD-Werte wie die Haupt-Stützstellen 18 bis 21 auf. Analog gilt dies auch für die Haupt-Stützstellen 26 bis 29 und 30 bis 33, die ebenfalls jeweils die gleichen preopACD-Werte aufweisen, jedoch an unterschiedlichen preopAL-Werten angeordnet sind.

Wie darüber hinaus aus der Darstellung in Fig. 4 zu erkennen ist, wird beispielsweise durch die Haupt-Stützstellen 20, 21, 22 und 23 ein erster Quadrant Q1 rechteckförmig aufgespannt, wobei die Haupt-Stützstellen 20 bis 23 die Eckpunkte dieses Rechtecks darstellen. Dies ist wieder im Hinblick auf die Projektion in die x-y-Ebene zu sehen (Fig. 4).

Die Haupt-Stützstellen 18 bis 33 sind so ausgewählt, dass die Größen der in die x-y-projizierten Teilnetze des Vorhersagenetzes 58 in Form der rechteckigen Quadranten Q1 bis Q9 in ihrer Flächengröße angepasst sind, und zwar an die jeweils in diesen Wertepaaren vorhandene Anzahl von Werten aus der Datenbasis der anderen Humanaugen.

Insbesondere ist vorgesehen, dass diese in Fig. 4 in die x-y-Ebene projizierten rechteckigen Quadranten Q1 und Q9 umso kleiner gestaltet sind, je größer die Werteanzahl der zugrundegelegten Datenbasis in diesen Flächenbereichen ist. Dadurch wird die Genauigkeit bei der weiteren Vorhersagebestimmung des postoperativen horizontalen Tiefenlagenwerts und somit eines Werts des Parameters postopeIOLE erhöht.

Wie zu erkennen ist, ist im Ausführungsbeispiel vorgesehen, dass das Vorhersagenetz 58 aus neun Teilnetzen bzw. Teilmaschen aufgespannt ist und somit insbesondere eine 3x3-Matrix erzeugt ist.

Die Anzahl der Teilnetze und somit insbesondere auch der in die x-y-projizierten ebenen Rechteckflächen mit den Quadranten Q1 bis Q9 ist daher lediglich beispielhaft. Es kann auch eine andere Anzahl von derartigen Teilnetzen vorgesehen sein und auch die Ausgestaltung als 3x3-Matrix ist lediglich beispielhaft.

Vorzugsweise ist vorgesehen, dass sich die Haupt-Stützstellen 18 bis 21 an einem Wert von preopAL = 15,00 mm befinden. Insbesondere ist im Ausführungsbeispiel vorgesehen, dass sich die Haupt-Stützstellen 22 bis 25 an einem Wert preopAL = 22,50 mm befinden. Insbesondere befinden sich die Haupt-Stützstellen 26 bis 29 an einem Wert preopAL = 25,00 mm. Des Weiteren befinden sich die Haupt-Stützstellen 30 bis 33 an einem Wert preopAL = 40,00 mm.

Im Ausführungsbeispiel ist darüber hinaus vorgesehen, dass sich die Haupt-Stützstellen 21, 22, 29 und 30 an einem Wert preopACD = 1,50 mm befinden. Des Weiteren ist insbesondere vorgesehen, dass sich die Haupt-Stützstellen 20, 23, 28 und 31 an einem Wert preopACD = 2,90 mm befinden. Darüber hinaus ist vorgesehen, dass sich die Haupt-Stützstellen 19, 24, 27 und 32 an einem Wert preopACD = 3,40 mm befinden. Insbesondere ist darüber hinaus vorgesehen, dass sich die Haupt-Stützstellen 18, 25, 26 und 33 an einem Wert preopACD = 6,00 mm befinden.

Aus diesen beispielhaften Werten lassen sich dann auch die Größen der projizierten Rechtecke bezüglich der Quadranten Q1 bis Q9 berechnen und insbesondere auch die Größenverhältnisse relativ zueinander zwischen den Quadranten Q1 und Q9 bestimmen.

Da für jeden der Haupt-Stützstellen 18 bis 33 auch die z-Werte und somit die Werte des Parameters postopeIOLE bekannt sind, kann hier für jede der Rand-Verbindungsgeraden 34 bis 57 eine Geradengleichung aufgestellt werden.

In der Tabelle 1 sind hierbei beispielhaft die oben genannten Werte der Haupt-Stützstellen 18 bis 33 für die Parameter preopACD, preopAL und als z-Wert die Werte des Parameters postopeIOLE eingetragen. Ausgehend davon lassen sich die in der Tabelle 1 dargestellten linearen Geradengleichungen für die Rand-Verbindungsgeraden 34 bis 57 berechnen. In der Tabelle 1 sind der Übersichtlichkeit dienend lediglich die linearen Gleichungen für die Rand-Verbindungsgeraden 34 bis 36, 41 bis 43, 48 bis 50 und 55 bis 57 dargestellt.

Im Hinblick auf die Nomenklatur bedeutet beispielsweise bei der Gleichung $Z_{RV\,36}^{x1}$ für die Rand-Verbindungs-gerade 36 das hochgestellte x1, das die Gerade für den konstanten Wert bzw. an der Stelle x1 des Parameters preopAL bestimmt wurde, wobei dies bei den beiden Haupt-Stützstellen 20 und 21, zwischen denen sich die Gleichung für die Rand-Verbindungsgeraden 36 erstreckt, bei dem Wert x1 = 15 ist. Die der Funktion z tiefgestellte Bezeichnung RV36 bezieht sich hierbei auf die Rand-Verbindungsgeraden 36. Diese erstreckt sich zwischen den Haupt-Stützstellen 20 und 21, so dass auch eine Nomenklatur HS 21-20 verwendet werden könnte.

Entsprechende Auslegung und Interpretation ist dann auch für die anderen in der Tabelle angegebenen linearen Gleichungen zu verstehen. Da beispielsweise die lineare Gleichung mit dem hochgestellten Index x2 und den tiefgestellten Index RV 43 sowohl eine Gleichung für eine Rand-Verbindungsgeraden 43 ist, die zum Quadranten Q1 als auch zum benachbarten und anschließenden Quadranten Q2 zugehörig ist, sind diese Gleichungen in der Tabelle 1 identisch.

Das Vorhersagenetz 58 kann abhängig von der Anzahl der ausgewählten Haupt-Stützstellen und der darauf resultierenden Teilmaschenanzahl beliebig feinmaschiger gestaltet werden.

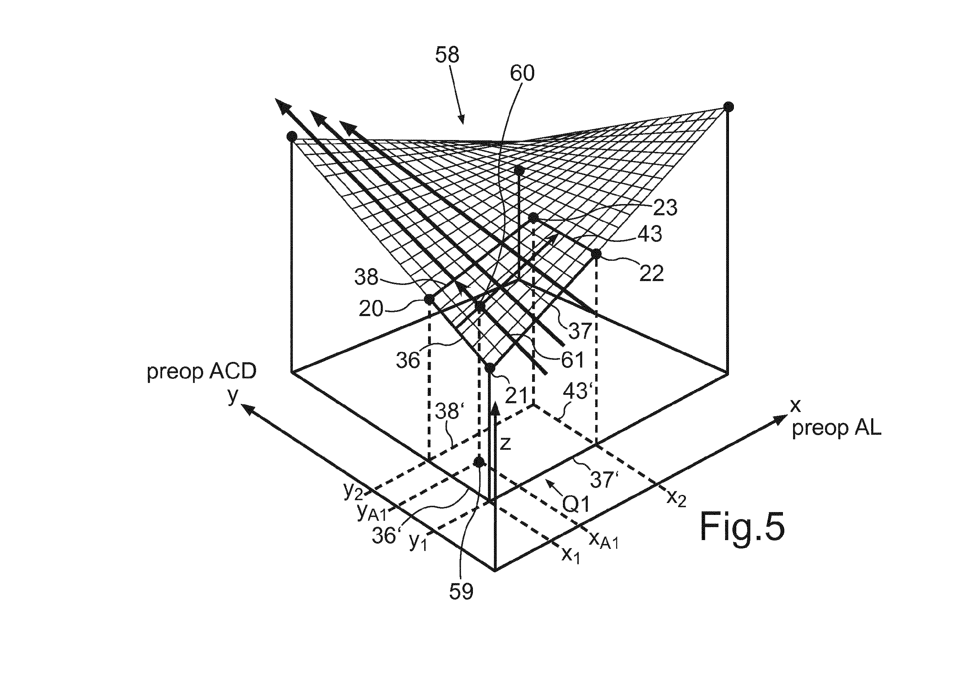

Ausgehend von dem aufgespannten Vorhersagenetz 58 wird dann im Weiteren gemäß der Darstellung in Fig. 5 eine Berechnung zur präoperativen Vorhersage eines Tiefenlagenwerts für die Parameter postopeIOLE für das erste Auge 1 durchgeführt.

Die in Fig. 3 gezeigte Darstellung des Vorhersagenetzes 58 kann unabhängig von dem ersten Auge erzeugt werden. Vielmehr wird es insbesondere ausschließlich auf gemessenen und/oder berechneten und/oder geschätzten Werten für die Vielzahl von unterschiedlichen anderen Augen und der damit zugrundegelegten Datenbasis erzeugt.

Die zwischen den Rand-Verbindungsgeraden gebildeten Flächen der Teilmaschen des Vorhersagenetzes 58 sind aufgrund der unterschiedlichen Orientierungen der jeweils zugrundegelegten Rand-Verbindungsgeraden, welche im gezeigten Ausführungsbeispiel die jeweiligen Begrenzungen der Teilnetze darstellen, unterschiedlich geneigte und orientierte Teilflächen im Raum. Dies kann insbesondere dahingehend verstanden werden, dass die jeweiligen Teilnetze und insbesondere die Rand-Verbindungsgeraden durch entsprechend aufgespannte Teilflächen verbunden werden. Aufgrund der unterschiedlichen Neigungen der Rand-Verbindungsgeraden ist jedoch üblicherweise eine Teilfläche eines Teilnetzes keine Ebene, sondern ebenfalls eine wiederum im dreidimensionalen Raum spezifisch gekrümmte Fläche.

Ausgehend von dem gemäß zu den Fig. 3 und Fig. 4 erläuterten Vorhersagenetz, welches im Ausführungsbeispiel das Vorhersagenetz 58 ist, kann dann im Weiteren für das spezifische zu untersuchende Auge 1 eine präoperative Vorhersage für den Tiefenlagenwert und somit einen Wert des Parameters postopeIOLE erfolgen.

Dabei wird gemäß der Darstellung in Fig. 5 zunächst das Wertepaar umfassend den präoperativ gemessenen Wert preopACD, welcher in der Darstellung gemäß Fig. 5 den Wert yA1 darstellt, und dem präoperativ gemessenen Wert preopAL des Auges 1, durch den der Wert xA1 gekennzeichnet ist, zugrundegelegt. Abhängig von diesen beiden Werten yA1 und xA1 wird dann derjenige Quadrant Q1 bis Q9 ausgewählt, durch welchen das Wertepaar dieser Parameter des Auges 1 umfasst ist. Beispielhaft ist im Ausführungsbeispiel vorgesehen, dass das Wertepaar yA1 und xA1 durch den Quadranten Q1 umfasst ist und somit im Vorhersagenetz 58 eine weitere Berechnung anhand der Teilmasche, das durch die Haupt-Stützstellen 20, 21, 22 und 23 an den Eckpunkten begrenzt ist, vorgenommen. Diese Teilmasche ist durch die Rand-Verbindungsgeraden 36, 37, 38 und 43 begrenzt.

Ausgehend von diesem Punkt 59 in der x-y-Ebene, der durch das Wertepaar yA1 und xA1 charakterisiert wird, werden dann im Weiteren Zwischen-Verbindungsgeraden erzeugt.

Diese sind wieder als lineare Gleichungen gegeben.

Dazu wird gemäß den nachfolgenden Formeln 1 und 2, die die Verallgemeinerungen der in der vorherigen Tabelle 1 für spezifische Werte und Ausführungsbeispiele beispielhaft angegebenen Formeln genannt sind, die Berechnung durchgeführt.

$$Z_{RV\,36}^{x1}(y)= m_{21|20}\, y + n_{21|20} \qquad\qquad (1)$$

$$Z_{RV\,43}^{x2}(y)= m_{22|23}\, y + n_{22|23} \qquad\qquad (2)$$

Die Geradengleichung für die Rand-Verbindungsgerade 36 ergibt sich dabei durch die Formel 1 und die Geradengleichung für die Rand-Verbindungsgerade 43 ergibt sich durch die Formel 2.

In diesen Formeln bezeichnen die Faktoren m mit ihren tiefgestellten Indizes jeweils die Steigung zwischen den beiden Haupt-Stützstellen 20 und 21 einerseits und den Haupt-Stützstellen 22 und 23 andererseits. y bezeichnet dabei den jeweiligen Wert entlang der y-Achse und charakterisiert somit einen Wert für den Parameter preopACD. Die Faktoren n mit ihren tiefgestellten Indizes charakterisieren dabei jeweils, wie es bei mathematischen Geradengleichungen üblich ist, den z-Wert, wenn für den Wert y = 0 eingesetzt wird.

Entsprechend stellen die Formeln 3 und 4 die allgemeinen Geradengleichungen für die Rand-Verbindungsgeraden 37 und 38 dar.

$$Z_{RV37}^{y1}(x) = m_{21|22}\, x + n_{21|22} \qquad (3)$$

$$Z_{RV38}^{y2}(x) = m_{20|23}\, x + n_{20|23} \qquad (4)$$

Die Gleichung gemäß Formel 3 ist dabei an dem konstanten y-Wert y1 und die Geradengleichung gemäß Formel 4 ist an dem konstanten y-Wert y2 bestimmt. y1 stellt dabei denjenigen y-Wert der Haupt-Stützstellen 21 und 22 dar, wohingegen y2 den y-Wert der Haupt-Stützstellen 20 und 23 darstellt.

Ausgehend von diesen vier Formeln wird dann eine erste Zwischen-Verbindungsgerade (ZV) 60 bestimmt. Dies erfolgt gemäß der nachfolgenden Formel 5.

$$Z_{ZV60}^{yA1}(x) = \frac{Z_{RV43}^{x2}(yA1) - Z_{RV36}^{x1}(yA1)}{x_2 - x_1} \cdot x + n_{ZV60} \qquad (5)$$

Bezüglich der Steigung dieser Geraden wird dabei eine Differenz zwischen den Werten der Formeln 1 und 2 an der Stelle yA1 bestimmt und dieser Wert zur Ermittlung der Steigung durch die Differenz der Werte x2 und x1 geteilt. n bezeichnet dabei wieder den z-Wert, wenn in die Formel 5 für den Wert x = 0 eingesetzt wird.

Die dann so ermittelte Formel 5 stellt dann die Gleichung für die erste Zwischen-Verbindungsgerade 60 dar. Bei der Projektion dieser Zwischen-Verbindungsgeraden 60 in die x-y-Ebene stellt diese eine Parallele zu den entsprechend in die x-y-Ebene projizierten Rand-Verbindungsgeraden 37' und 38' dar.

Wird in diese Gleichung gemäß Formel 5 dann der gemessene Wert xA1 des Auges 1 eingesetzt, so erhält man den z-Wert dieses Auges 1, der dann auch als präoperativer Vorhersagewert für eine postoperative horizontale Tiefenlage postopeIOLE der Intraokularlinse im Auge 1 darstellt.

Zur Verbesserung der präoperativen Vorhersage kann insbesondere zusätzlich vorgesehen sein, dass eine zweite Zwischen-Verbindungsgerade 61 bestimmt wird. Diese wird gemäß der nachfolgenden Formel 6 berechnet, wobei hier wiederum die Steigung, die dann mit y multipliziert wird, analog zur Formel 5 berechnet wird, wobei hier die Differenz der Werte der Formeln 3 und 4 an dem Wert xA1 berechnet wird und durch die Differenz der Werte y2 - y1 dividiert wird.

$$Z_{ZV61}^{xA1}(y) = \frac{Z_{RV38}^{y2}(xA1) - Z_{RV37}^{y1}(xA1)}{y_2 - y_1} \cdot y + n_{ZV61} \qquad (6)$$

Ausgehend von der Formel 6 kann dann dort durch Einsetzen des Wertes yA1 der z-Wert der zweiten Zwischen-Verbindungsgrad 61 bestimmt werden.

Dies ist dahingehend vorteilhaft, da aufgrund der Formgestaltung der dreidimensionalen Teilmasche des Quadranten Q1, die durch die Rand-Verbindungsgeraden 36, 37, 38 und 43 begrenzt ist, sich die beiden Zwischen-Verbindungsgeraden 60 und 61 an der Stelle xA1 und yA1 nicht schneiden, was bedeutet, dass die daraus resultierenden z-Werte unterschiedlich sind.

Um nun ein bestmögliches und präzises Vorhersageergebnis für den Wert des Parameters postopeIOLE zu erhalten, kann dann abhängig von diesen beiden z-Werten der Zwischen-Verbindungsgeraden 60 und 61 weiterhin unterschiedlich verfahren werden. So kann beispielsweise vorgesehen sein, dass die Differenz dieser beiden z-Werte gemittelt wird, wobei insbesondere diese Werte dann voneinander abgezogen werden und der Betrag dieser Differenz durch zwei geteilt wird. Insbesondere kann dann vorgesehen sein, dass der gemittelte Differenzwert zu dem kleineren der beiden z-Werte, die sich aus Zwischen-Verbindungsgeraden ergeben, addiert wird. Ebenso kann jedoch auch vorgesehen sein, dass dieser gemittelte Differenzwert von dem größeren der beiden z-Werte, die sich aus den Zwischen-Verbindungsgeraden ergeben, abgezogen wird.

Die Vorgehensweise, wie verfahren wird, wenn sich zwei verschiedene z-Werte aus den Zwischen-Verbindungsgeraden ergeben, um dann eine möglichst präzise Aussage für die präoperative Vorhersage der postoperativen horizontalen Tiefenlage der Intraokularlinse im Auge zu treffen, kann dann von vielerlei Gegebenheiten und Parametern abhängen. So kann dies beispielsweise abhängig von der Größe des Differenzwerts erfolgen und/oder abhängig von den spezifischen Quadranten Q1 bis Q9, in dem sich das Wertepaar xA1, yA1 befindet und/oder abhängig von der Größe und/oder Lage des Quadranten Q1 bis Q9, in dem sich dieses Wertepaar befindet, sein. Die Aussagen bezüglich des Quadranten gelten natürlich in analoger Weise auch für die im dreidimensionalen Raum sich befindlichen Teilflächen beziehungsweise Teilnetze des Vorhersagenetzes 58, die das Wertepaar xA1, yA1 abdecken.

Diesbezüglich wird dann dieser vorhergesagte z-Wert und somit der Wert der postoperativen horizontalen Tiefenlage postopeIOLE der Intraokularlinse in diesem ersten Auge 1 berechnet. Er wird dann als weiterer Wert, der wesentlich ist, in die Berechnung der Brechkraft der zu implantierenden Intraokularlinse in das erste Auge 1 berücksichtigt.

Die eben genannten Erläuterungen basieren auf der Angabe, dass die Intraokularlinse 14 gemäß der Darstellung in Fig. 2 Haptiken 62 und 63 aufweist, die gegenüber der Vertikalen nicht nach vorne oder nach hinten geneigt sind. Darüber hinaus ist vorgesehen, dass die Intraokularlinse 14 in dem Kapselsack 3 des Auges 1 implantiert werden soll.

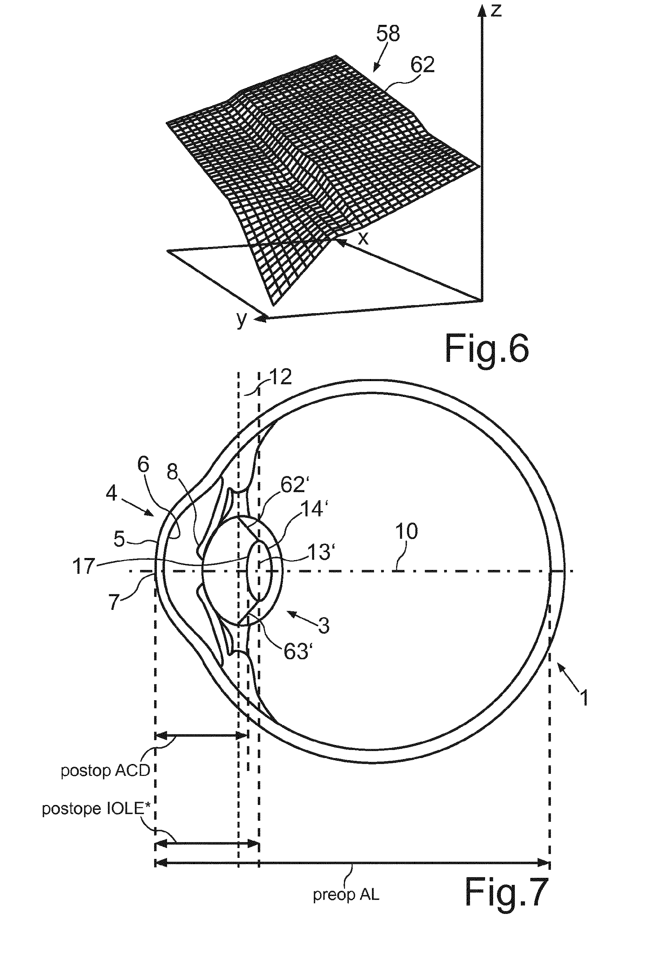

In Fig. 6 ist eine beispielhafte Darstellung eines uneben ausgebildeten Vorhersagenetzes 58 gezeigt, welches in dem dreidimensionalen Koordinatensystem angeordnet ist. Die unterschiedlichen Neigungen und Krümmungen sind zu erkennen. Es kann auch vorgesehen sein, dass das Vorhersagenetz 58, beispielsweise durch Interpolation ausgehend von den bereitgestellten Rand-Verbindungsgeraden eine vollständig zusammenhängende Vorhersagefläche bildet. Diese kann dann gemäß der Vorhersagefläche 62 in Fig. 6 ausgestaltet sein.

Ist eine derartige zusammenhänge Vorhersagefläche 62 erzeugt, so kann auch vorgesehen sein, dass abhängig von den gemessenen Werten xA1 und yA1 keine weitere Berechnung von Zwischen-Verbindungsgeraden und gegebenenfalls zu ermittelnden z-Werten der Zwischen-Verbindungsgeraden durchgeführt werden muss, sondern dass dann explizit aus der vorhandenen Vorhersagefläche 62 schon ein einziger z-Wert entnommen werden kann. Diesbezüglich ist der Rechenaufwand für die Bestimmung des z-Werts niedriger, nachdem bereits das Vorhersagenetz 58 und daraus folgend die Vorhersagefläche 62 bestimmt wurde. Allerdings ist im Vergleich zur Vorhergehensweise, wie sie zu den Fig. 3 bis Fig. 5 dargelegt wurde, ein größerer vorhergehender Rechenaufwand erforderlich, um überhaupt die Vorhersagefläche 62 entsprechend genau und präzise bestimmen und bereitstellen zu können. Insbesondere ist es dabei vorgesehen, dass eine sehr hohe Anzahl an Haupt-Stützstellen bereitgestellt wird, um ein besonders engmaschiges Vorhersagenetz 58 generieren zu können, welches dann beispielsweise durch Interpolation eine entsprechende exakt und uneben geformte Vorhersagefläche 62 ermöglicht.

Ausgehend von den Erläuterungen zur spezifischen Ausgestaltung in Fig. 2 wird dann die so simulativ ermittelte vorhergesagte Tiefenlage und somit der präoperativ vorhergesagte postoperative horizontale Tiefenlagewert der Intraokularlinse im Auge 1 modelliert und daraus dann die Brechkraft und die Form der Intraokularlinse 14 modelliert. Die dann so auf Basis dieser Modellierungswerte hergestellte Intraokularlinse 14 wird dann in das Auge 1 entsprechend implantiert. Es kann dann durch Messen des in Fig. 2 eingezeichneten Parameterwerts des Parameters postopACD die vorhergesagte Lage der Intraokularlinse 14 überprüft werden. In Fig. 2 ist beispielhaft der dann bereits implantierte Zustand der Intraokularlinse 14 in dem Simulationsmodell des Auges 1 eingezeichnet.

In einer weiteren Ausführung kann vorgesehen sein, dass die Intraokularlinse 14' gegenüber der Vertikalen nach vorne gewinkelt und somit geneigte Haptiken 62' und 63' aufweist, wie dies in Fig. 7 gezeigt ist. Um auch für derartige Konstellationen entsprechend präzise Vorhersagen für den postoperativen horizontalen Tiefenlagenwert postopeIOLE* ermöglichen zu können, sind die oben genannten und dargelegten Formeln 5 und 6 zu modifizieren. Insbesondere ist dabei dann eine Formel 7 und gegebenenfalls 8, wie sie nachfolgend dargestellt sind, heranzuziehen.

$$Z_{ZV60}^{yA1}(x)* = Z_{ZV60}^{yA1}(x) - 1{,}39329 + \left[\left(Fak_Z \cdot 0{,}5663\right) - 65{,}60\right] \quad (7)$$

$$Z_{ZV61}^{xA1}(y)* = Z_{ZV61}^{xA1}(y) - 1{,}39329 + \left[\left(Fak_Z \cdot 0{,}5663\right) - 65{,}60\right] \quad (8)$$

In dieser sind die unterschiedlichen Gegebenheiten mit einer angewinkelten Haptik der Intraokularlinse und/oder eine Implantation mit dem Kapselsack des Auges 1 berücksichtigt.

Der Z-Faktor $Fak_Z$ kann ein statischer vorgegebener Wert sein, der abhängig von der Linsenart und der Linsenform IOL sowie der Anbringung an spezifischer Stelle im Auge variiert. Insbesondere kann dieser Wert zwischen 110 und 125 variieren.

Besonders vorteilhaft ist es, wenn dieser Faktor $Fak_Z$ selbst wiederum eine Funktion ist, die insbesondere von einem spezifischen Parameter der vorgesehenen Intraokularlinse abhängt, insbesondere von der Brechkraft abhängig ist. Insbesondere könnte hier vorzugsweise eine lineare Funktion zugrunde gelegt werden.

Bezüglich der erläuterten Vorgehensweise zu Fig. 5 kann zunächst auch die Zwischen-Verbindungsgeraden 61 bestimmt werden und für die z-Wertbestimmung und somit den Wert postopeIOLE alleine genügen. Optional kann dann nachfolgend die Zwischen-Verbindungsgeraden 60 bestimmt werden und das weitere Vorgehen wie in Fig. 5 erläutert erfolgen. Entsprechendes gilt für die Formeln 7 und 8.

**Patentansprüche**

1. Verfahren zur präoperativen Vorhersage einer postoperativen horizontalen Tiefenlage (postopeIOLE, postopeIOLE*) einer Intraokularlinse (14, 14') in einem Patientenauge (1), bei welchem Werte von Parametern bereitgestellt werden, wobei die Parameter einen ersten Parameter als Tiefe (preopACD) einer vorderen Augenkammer (7) von Humanaugen, einen zweiten Parameter als horizontale Länge (preopAL) von Humanaugen und einen dritten Parameter als postoperative Lage von Intraokularlinsen in Humanaugen umfassen,
**dadurch gekennzeichnet, dass**
aus diesen Werten spezifische Wertetripel mit jeweils einem Wert der drei genannten Parameter als Haupt-Stützstellen (18 bis 33) ausgewählt werden und die Haupt-Stützstellen (18 bis 33) in ein durch die drei Parameter aufgespanntes dreidimensionales Koordinatensystem eingetragen werden, wobei Haupt-Stützstellen (18 bis 33) durch Rand-Verbindungsgeraden (34 bis 57) verbunden werden und zumindest durch die Lagen der Rand-Verbindungsgeraden (34 bis 57) ein im dreidimensionalen Koordinatensystem uneben geformtes Vorhersagenetz (58) zur Vorhersage der postoperativen horizontale Tiefenlage (postopeIOLE, postopeIOLE*) der Intraokularlinse (14, 14') im Patientenauge (1) erzeugt wird, wobei Parameter des Patientenauges (1) gemessen werden und für die Vorhersage der Tiefenlage bereitgestellt werden und abhängig von den gemessenen Parametern des Patientenauges (1) und von dem Vorhersagenetz (58) die postoperative horizontale Tiefenlage (postopeIOLE, postopeIOLE*) bestimmt und bereitgestellt wird.

2. Verfahren nach Anspruch 1,
**dadurch gekennzeichnet, dass**
die präoperativ vorherzusagende postoperative horizontale Tiefenlage (postopeIOLE, postopeIOLE*) der Intraokularlinse (14, 14') im Patientenauge (1) abhängig von zumindest einer zur Erzeugung des Vorhersagenetzes (58) beitragenden Verbindungsgeraden (34 bis 57, 60, 61) bestimmt wird.

3. Verfahren nach Anspruch 1 oder 2,
**dadurch gekennzeichnet, dass**
das Vorhersagenetz (58) aus einer Mehrzahl von Teilmaschen erzeugt wird, und die Haupt-Stützstellen (18 bis 33) so gewählt werden, dass sie jeweils Eckpunkte zumindest einer Teilmasche sind, insbesondere die Haupt-Stützstellen (18 bis 33) so ausgewählt werden, dass die Teilmaschen bei einer Projektion der sie begrenzenden Rand-Verbindungsgeraden (34 bis 57) in die Koordinatenebene, welche durch die senkrecht zueinander stehenden Koordinatenachsen für den Parameter der Tiefe (preopACD) der vorderen Augenkammer (7) und den Parameter der Länge (preopAL) des Humanauges aufgespannt wird, als Rechtecke aufgespannt werden und Quadranten (Q1 bis Q9) der Gesamtfläche bilden, insbesondere die Rechtecke so in das Koordinatensystem eingebracht werden, dass die Rand-Verbindungsgeraden (34 bis 57) zwischen jeweils zwei Eckpunkten einer Teilmasche bei einer Projektion der Rand-Verbindungsgeraden (34 bis 57) in die Koordinatenebene, welche durch die senkrecht zueinander stehenden Koordinatenachsen für den Parameter der Tiefe (preo-pACD) der vorderen Augenkammer (7) und den Parameter der Länge (preopAL) des Humanauges aufgespannt wird, parallel zu den Koordinatenachsen verlaufen.

4. Verfahren nach einem der vorhergehenden Ansprüche,
**dadurch gekennzeichnet, dass**
die Haupt-Stützstellen (18 bis 33) so ausgewählt werden, dass die Längen der in eine durch die mit den Parametern der Tiefe der vorderen Augenkammer und der Länge des Auges charakterisierten Koordinatenachsen aufgespannten Koordinatenebene projizierten Rand-Verbindungsgeraden (34 bis 57) der Quadranten (Q1 bis Q9) in der Koordinatenebene in Richtung der ersten Koordinatenachse (preopACD, peropAL) unterschiedlich erzeugt werden und/oder die Haupt-Stützstellen (18 bis 33) so ausgewählt werden, dass die Längen der in eine durch die mit den Parametern der Tiefe der vorderen Augenkammer und der Länge des Auges charakterisierten Koordinatenachsen aufgespannten Koordinatenebene projizierten Rand-Verbindungsgeraden (34 bis 57) von zumindest zwei Quadranten (Q1 bis Q9) in der Koordinatenebene in Richtung der zweiten Koordinatenachse (preopACD, preopAL) unterschiedlich erzeugt werden.

5. Verfahren nach einem der vorhergehenden Ansprüche,
**dadurch gekennzeichnet, dass**
abhängig von dem Wertepaar der Tiefe (preopACD) der vorderen Augenkammer (7) des Patientenauges (1) und der Länge (preopAL) des Patientenauges (1) diejenige Teilmasche mit dem zugeordneten Quadranten (Q1 bis Q9) des Vorhersagenetzes (58) ausgewählt wird, durch welche das Wertepaar in den Koordinatenachsen (preo-pACD, preopAL) umfasst wird.

**6.** Verfahren nach Anspruch 5,
**dadurch gekennzeichnet, dass**
für den Wert der Tiefe (preopACD) der vorderen Augenkammer (7) des Patientenauges (1) eine erste zur Erzeugung des Vorhersagenetzes (58) beitragende Zwischen-Verbindungsgerade (60, 61) abhängig von den Werten der Rand-Verbindungsgeraden (34 bis 57) der ausgewählten Teilmasche an der Stelle des Werts der Tiefe (preopACD) der vorderen Augenkammer (7) des Patientenauges (1) bestimmt wird und/oder für den Wert der Länge (preopAL) des Patientenauges (1) eine zweite zur Erzeugung des Vorhersagenetzes (58) beitragende Zwischen-Verbindungsgerade (60, 61) abhängig von den Werten der Rand-Verbindungsgeraden (34 bis 57) der ausgewählten Teilmasche an der Stelle des Werts der Länge (preopAL) des Patientenauges (1) bestimmt wird.

**7.** Verfahren nach Anspruch 6,
**dadurch gekennzeichnet, dass**
an der Stelle des Wertepaars der Tiefe (preopACD) der vorderen Augenkammer (7) und der Länge (preopAL) des Patientenauges (1) zumindest ein Vorhersagewert der postoperativen Tiefenlage (postopeIOLE, postopeIOLE*) der Intraokularlinse (14, 14') im Patientenauge (1) aus zumindest einer Zwischen-Verbindungsgeraden (60, 61) berechnet wird, insbesondere aus beiden Zwischen-Verbindungsgeraden (60, 61) jeweils ein Vorhersagewert berechnet wird und die beiden Vorhersagewerte verglichen werden und abhängig von einer auftretenden Differenz der Vorhersagewerte die weitere Bestimmungsart eines End-Vorhersagewerts der postoperativen Tiefenlage (postopACD, postopeIOLE) der Intraokularlinse (14, 14') im Patientenauge (1) zur präoperativen Vorhersage festgelegt wird, insbesondere eine Mittelwertbestimmung festgelegt wird.

**8.** Verfahren nach einem der vorhergehenden Ansprüche,
**dadurch gekennzeichnet, dass**
für die Bestimmung des postoperativen Werts der Tiefenlage (postopeIOLE, postopeIOLE*) der Intraokularlinse (14, 14') im Patientenauge (1) für eine vorgesehene Intraokularlinse (14, 14') mit einer gegenüber der Vertikalen geneigten Haptik (62, 63, 62', 63') und/oder für einen spezifischen vorgesehenen Implantationsort der Intraokularlinse (14, 14') im Patientenauge (1) ein erster Korrekturfaktor berücksichtigt wird, insbesondere ein Korrekturfaktor berücksichtigt wird, der abhängig von der Linsenform und/oder des Implantationsorts im Patientenauge (1) variabel bestimmt wird.

**9.** Verfahren nach Anspruch 8,
**dadurch gekennzeichnet, dass**
der Korrekturfaktor (Fak$_{korr}$) durch folgenden funktionellen Zusammenhang bestimmt wird:

$$Fak_{korr} = -a + \left[ \left( Fak_{Z} \cdot b \right) - c \right];$$

wobei a zwischen 1,3 und 1,43, b zwischen 0,45 und 0,65 und c zwischen 60 und 70, vorzugsweise zwischen 64 und 66 liegt,
insbesondere gilt:

$$Fak_{korr} = -1,39329 + \left[ \left( Fak_{Z} \cdot 0,5663 \right) - 65,60 \right],$$

und insbesondere der Faktor FakZ zwischen 110 und 125 liegt und/oder der Faktor FakZ als eine Funktion, insbesondere eine lineare Funktion, abhängig von einem Parameter der Intraokularlinse (14, 14'), insbesondere deren Brechkraft, festgelegt wird.

**10.** Verfahren nach einem der vorhergehenden Ansprüche,
**dadurch gekennzeichnet, dass**
die zum Auswählen der Haupt-Stützstellen (18 bis 33) bereitgestellten Werte der Tiefen von vorderen Augenkammern von Humanaugen durch Messen von Humanaugen bestimmt werden und/oder Werte von Längen von Humanaugen durch Messen von Humanaugen bestimmt werden und/oder Werte von postoperativen Tiefenlagen von Intraokularlinsen in Humanaugen gemessen und/oder geschätzt und/oder berechnet werden, insbesondere gemessene Werte nur bei Haupt-Stützstellen (18 bis 33) berücksichtigt werden, die mit das Versorgungsnetz (58) umfangsseitig begrenzenden Rand-Verbindungsgeraden (34 bis 57) verbunden werden.

**11.** Verfahren nach einem der vorhergehenden Ansprüche,
**dadurch gekennzeichnet, dass**
die Haupt-Stützstellen (18 bis 33) nutzerdefiniert vorgegeben werden.

**12.** Verfahren nach einem der vorhergehenden Ansprüche,,
**dadurch gekennzeichnet, dass**
das Vorhersagenetz (58) und/oder die bestimmte und präoperativ vorhergesagte postoperative horizontale Tiefenlage (postopeIOLE, postopeIOLE*) der Intraokularlinse (14, 14') auf einer Anzeigeeinheit einer Vorrichtung angezeigt werden, insbesondere die bestimmte und präoperativ vorhergesagte postoperative horizontale Tiefenlage (postopeIOLE, postopeIOLE*) der Intraokularlinse (14, 14') auf einer Anzeigeeinheit einer Vorrichtung als Wert und/oder in einem als Bild dargestellten Auge angezeigt werden.

**13.** Verfahren nach einem der vorhergehenden Ansprüche,
**dadurch gekennzeichnet, dass**
abhängig von der präoperativ vorhergesagten postoperativen horizontalen Tiefenlage (postopeIOLE, postopeIOLE*) der Intraokularlinse (14, 14') in dem Patientenauge (1) die Intraokularlinse (14, 14') in ihrer Form und/oder Brechkraft modelliert wird.

**14.** Verfahren nach einem der vorhergehenden Ansprüche,
**dadurch gekennzeichnet, dass**
ein einmal erzeugtes Vorhersagenetz (58) abhängig von nachträglich erhaltenen Informationen über die tatsächliche positionelle Tiefenlage der Intraokularlinse (14, 14') im Patientenauge (1) nach deren Implantation korrigiert wird und insbesondere entsprechende Haupt-Stützstellen (18 bis 33), insbesondere automatisch, verändert werden.

**15.** Vorrichtung zur Durchführung eines Verfahrens nach einem der vorhergehenden Ansprüche, welche zumindest ein Mittel aufweist, um das Vorhersagenetz (58) zu bestimmen, und zumindest ein Mittel umfasst, um den Vorhersagewert zu bestimmen.

**Claims**

**1.** Method for preoperative prediction of a postoperative horizontal depth (postopeIOLE, postopeIOLE*) of an intraocular lens (14, 14') in a patient's eye (1), in which values of parameters are provided, wherein the parameters include a first parameter as the depth (preopACD) of an anterior chamber (7) of human eyes, a second parameter as the horizontal length (preopAL) of human eyes and a third parameter as the postoperative position of intraocular lenses in human eyes,
**characterized in that**
specific value triplets with each one value of the three mentioned parameters are selected from these values as main interpolation nodes (18 to 33) and the main interpolation nodes (18 to 33) are entered into a three-dimensional coordinate system spanned by the three parameters, wherein main interpolation nodes (18 to 33) are connected by edge connecting lines (34 to 57), and at least by the positions of the edge connecting lines (34 to 57), a predictive network (58) unevenly shaped in the three-dimensional coordinate system for predicting the postoperative horizontal depth (postopeIOLE, postopeIOLE*) of the intraocular lens (14, 14') in the patient's eye (1) is generated, wherein parameters of the patient's eye (1) are measured and provided for the prediction of the depth, and depending on the measured parameters of the patient's eye (1) and on the predictive network (58), the postoperative horizontal depth (postopeIOLE, postopeIOLE*) is determined and provided.

**2.** Method according to claim 1,
**characterized in that**
the postoperative horizontal depth (postopeIOLE, postopeIOLE*) of the intraocular lens (14, 14') in the patient's eye (1) to be preoperatively predicted is determined depending on at least one connecting line (34 to 57, 60, 61) contributing to the generation of the predictive network (58).

**3.** Method according to claim 1 or 2,
**characterized in that**
the predictive network (58) is generated from a plurality of partial meshes, and the main interpolation nodes (18 to 33) are selected such that they each are vertices of at least one partial mesh, in particular the main interpolation nodes (18 to 33) are selected such that the partial meshes are spanned as rectangles upon projection of the edge connecting lines (34 to 57) bounding them into the coordinate plane, which is spanned by the mutually perpendicular coordinate axes for the parameter of the depth (preopACD) of the anterior chamber (7) and the parameter of the length (preopAL) of the human eye, and constitute quadrants (Q1 to Q9) of the overall area, in particular the rectangles are introduced into the coordinate system such that the edge connecting lines (34 to 57) extend parallel to the coordinate axes between each two vertices of a partial mesh upon projection of the edge connecting lines (34 to 57) into the coordinate plane, which is spanned by the mutually perpendicular coordinate axes for the parameter of the depth (preopACD) of the anterior chamber (7) and the parameter of the length (preopAL) of the human eye.

4.  Method according to any one of the preceding claims,
    **characterized in that**
    the main interpolation nodes (18 to 33) are selected such that the lengths of the edge connecting lines (34 to 57) of the quadrants (Q1 to Q9) projected into a coordinate plane spanned by the coordinate axes **characterized by** the parameters of the depth of the anterior chamber and the length of the eye are differently generated in the direction of the first coordinate axis (preopACD, preopAL) in the coordinate plane and/or the main interpolation nodes (18 to 33) are selected such that the lengths of the edge connecting lines (34 to 57) of at least two quadrants (Q1 to Q9) projected into a coordinate plane spanned by the coordinate axes **characterized by** the parameters of the depth of the anterior chamber and the length of the eye are differently generated in the direction of the second coordinate axis (preopACD, preopAL) in the coordinate plane.

5.  Method according to any one of the preceding claims,
    **characterized in that**
    depending on the pair of values of the depth (preopACD) of the anterior chamber (7) of the patient's eye (1) and the length (preopAL) of the patient's eye (1), that partial mesh with the associated quadrant (Q1 to Q9) of the predictive network (58) is selected, by which the pair of values in the coordinate axes (preopACD, preopAL) is encompassed.

6.  Method according to claim 5,
    **characterized in that**
    for the value of the depth (preopACD) of the anterior chamber (7) of the patient's eye (1), a first intermediate connecting line (60, 61) contributing to the generation of the predictive network (58) is determined depending on the values of the edge connecting lines (34 to 57) of the selected partial mesh at the location of the value of the depth (preopACD) of the anterior chamber (7) of the patient's eye (1), and/or for the value of the length (preopAL) of the patient's eye (1), a second intermediate connecting line (60, 61) contributing to the generation of the predictive network (58) is determined depending on the values of the edge connecting lines (34 to 57) of the selected partial mesh at the location of the value of the length (preopAL) of the patient's eye (1).

7.  Method according to claim 6,
    **characterized in that**
    at the location of the pair of values of the depth (preopACD) of the anterior chamber (7) and the length (preopAL) of the patient's eye (1), at least one predictive value of the postoperative depth (postopelOLE, postopelOLE*) of the intraocular lens (14, 14') in the patient's eye (1) is calculated from at least one intermediate connecting line (60, 61), in particular each one predictive value is calculated from both intermediate connecting lines (60, 61), and the two predictive values are compared, and depending on an occurring difference of the predictive values, the further mode of determination of a final predictive value of the postoperative depth (postopACD, postopelOLE) of the intraocular lens (14, 14') in the patient's eye (1) is determined for preoperative prediction, in particular an average determination is determined.

8.  Method according to any one of the preceding claims,
    **characterized in that**
    for the determination of the postoperative value of the depth (postopelOLE, postopelOLE*) of the intraocular lens (14, 14') in the patient's eye (1) for a provided intraocular lens (14, 14') with a haptic (62, 63, 62', 63') inclined with respect to the vertical and/or for a specific provided implantation location of the intraocular lens (14, 14') in the patient's eye (1), a first correction factor is taken into account, in particular a correction factor is taken into account, which is variably determined depending on the lens shape and/or the implantation location in the patient's eye (1).

9.  Method according to claim 8,
    **characterized in that**
    the correction factor ($Fak_{korr}$) is determined by the following functional correlation:

$$Fak_{korr} = -a + \left[(Fak_Z \cdot b) - c\right];$$

wherein a is between 1.3 and 1.43, b is between 0.45 and 0.65 and c is between 60 and 70, preferably between 64 and 66,
in particular, it applies:

$$Fak_{korr} = -1{,}39329 + \left[(Fak_Z \cdot 0{,}5663) - 65{,}60\right],$$

and in particular the factor FakZ is between 110 and 125 and/or the factor FakZ is determined as a function, in particular a linear function, depending on a parameter of the intraocular lens (14, 14'), in particular the refractive power thereof.

10. Method according to any one of the preceding claims,
**characterized in that**
the values of the depths of anterior chambers of human eyes provided for selecting the main interpolation nodes (18 to 33) are determined by measuring human eyes and/or values of lengths of human eyes are determined by measuring human eyes and/or values of postoperative depths of intraocular lenses in human eyes are measured and/or estimated and/or calculated, in particular measured values are only taken into account at main interpolation nodes (18 to 33), which are connected to edge connecting lines (34 to 57) circumferentially bounding the predictive network (58).

11. Method according to any one of the preceding claims,
**characterized in that**
the main interpolation nodes (18 to 33) are preset in user-defined manner.

12. Method according to any one of the preceding claims,
**characterized in that**
the predictive network (58) and/or the determined and preoperatively predicted postoperative horizontal depth (postopeIOLE, postopeIOLE*) of the intraocular lens (14, 14') is displayed on a display unit of a device, in particular the determined and preoperatively predicted postoperative horizontal depth (postopeIOLE, postopeIOLE*) of the intraocular lens (14, 14') is displayed on a display unit of a device as a value and/or in an eye presented as an image.

13. Method according to any one of the preceding claims,
**characterized in that**
depending on the preoperatively predicted postoperative horizontal depth (postopeIOLE, postopeIOLE*) of the intraocular lens (14, 14') in the patient's eye (1), the intraocular lens (14, 14') is modeled in its shape and/or refractive power.

14. Method according to any one of the preceding claims,
**characterized in that**
a once generated predictive network (58) is corrected depending on subsequently obtained information about the actual positional depth of the intraocular lens (14, 14') in the patient's eye (1) after implantation thereof and in particular corresponding main interpolation nodes (18 to 33) are in particular automatically changed.

15. Device for performing a method according to any one of the preceding claims, which has at least a means in order to determine the predictive network (58) and includes at least a means to determine the predictive value.

**Revendications**

1. Procédé destiné à prévoir avant l'opération une position en profondeur horizontale, postopératoire (postopeIOLE, postopeIOLE*) d'une lentille intraoculaire (14, 14') dans l'oeil (1) d'un patient, pour lequel des valeurs de paramètres sont produites, moyennant quoi les paramètres comprennent un premier paramètre comme profondeur (preopACD) d'une chambre antérieure (7) d'yeux humains, un deuxième paramètre comme longueur horizontale (preopAL) d'yeux humains et un troisième paramètre comme position postopératoire de lentilles intraoculaires dans des yeux humains,

**caractérisé en ce que**,

à partir de ces valeurs, des valeurs triples spécifiques, avec respectivement une valeur des trois paramètres nommés, sont sélectionnées comme points d'appui principaux (18 à 33) et les points d'appui principaux (18 à 33) sont intégrés dans un système de coordonnées en trois dimensions défini par les trois paramètres, moyennant quoi les points d'appui principaux (18 à 33) sont reliés par des droites de liaison de bords (34 à 57) et un réseau (58) prévisionnel, formé de manière non plane dans le système de coordonnées en trois dimensions, destiné à prévoir la position en profondeur horizontale, postopératoire (postopeIOLE, postopeIOLE*) de la lentille intraoculaire (14, 14') dans l'oeil (1) du patient, est généré au moins par les positions des droites de liaison de bords (34 à 57), moyennant quoi les paramètres de l'oeil (1) du patient sont mesurés et produits pour la prévision de la position en profondeur et la position en profondeur horizontale, postopératoire (postopeIOLE, postopeIOLE*) déterminée et produite en fonction des paramètres mesurés de l'oeil (1) du patient et du réseau prévisionnel (58).

2.  Procédé selon la revendication 1,
    **caractérisé en ce que**
    la position en profondeur horizontale, postopératoire, à prévoir avant l'opération (postopeIOLE, postopeIOLE*) de la lentille intraoculaire (14, 14') dans l'oeil (1) du patient est déterminée en fonction d'au moins l'une des droites de liaison (34 à 57, 60, 61) qui contribue à générer le réseau prévisionnel (58).

3.  Procédé selon la revendication 1 ou 2,
    **caractérisé en ce que**
    le réseau prévisionnel (58) est généré à partir d'une pluralité de maillages partiels et les points d'appui principaux (18 à 33) sont sélectionnés de telle sorte qu'ils représentent respectivement des points d'ancrage d'au moins un maillage partiel, les points d'appui principaux (18 à 33) sont sélectionnés en particulier de telle sorte que les maillages partiels sont définis, en cas de projection des droites de liaison de bords (34 à 57) qui les délimitent, dans le plan de coordonnées, qui est défini par les axes des coordonnées disposés verticalement les uns par rapport aux autres du paramètre de la profondeur (preopACD) de la chambre antérieure (7) de l'oeil et du paramètre de la longueur (preopAL) de l'oeil humain, comme des rectangles et constituent des quadrants (Q1 à Q9) de l'ensemble de la surface, les rectangles sont intégrés en particulier dans le système de coordonnées, de telle sorte que les droites de liaison de bords (34 à 57) évoluent parallèlement aux axes des coordonnées entre respectivement deux points d'ancrage d'un maillage partiel, en cas de projection des droites de liaison de bords (34 à 57) dans le plan des coordonnées, qui est défini par les axes des coordonnées, disposés verticalement les uns par rapport aux autres, du paramètre de la profondeur (preopACD), de la chambre antérieure (7) de l'oeil et du paramètre de la longueur (preopAL) de l'oeil humain.

4.  Procédé selon l'une des revendications précédentes,
    **caractérisé en ce que**
    les points d'appui principaux (18 à 33) sont sélectionnés de telle sorte que les longueurs des droites de liaison de bords (34 à 57), projetées dans un plan des coordonnées, défini par les axes des coordonnées, **caractérisés par** les paramètres de la profondeur de la chambre antérieure de l'oeil et de la longueur de l'oeil, des quadrants (Q1 à Q9), sont générées différemment dans le plan des coordonnées en direction du premier axe de coordonnées (preopACD, preopAL) et / ou que les points d'appui principaux (18 à 33) sont sélectionnés de telle sorte que les longueurs des droites de liaison de bords (34 à 57), projetées dans un plan des coordonnées, défini par les axes de coordonnées, **caractérisés par** les paramètres de la profondeur de la chambre antérieure de l'oeil et de la longueur de l'oeil, sont générées différemment par au moins deux quadrants (Q1 à Q9) dans le plan des coordonnées en direction du deuxième axe des coordonnées (preopACD, preopAL).

5.  Procédé selon l'une des revendications précédentes,
    **caractérisé en ce que**
    c'est le maillage partiel avec le quadrant (Q1 à Q9) affecté du réseau prévisionnel (58) qui est sélectionné, en fonction de la paire de valeurs de la profondeur (preopACD) de la chambre antérieure (7) de l'oeil (1) du patient et de la longueur (preopAL) de l'oeil (1) du patient, par le biais duquel la paire de valeurs est comprise dans les axes des coordonnées (preopACD, preopAL).

6.  Procédé selon la revendication 5,
    **caractérisé en ce que**,
    pour la valeur de la profondeur (preopACD) de la chambre antérieure (7) de l'oeil (1) du patient, une première droite de liaison intermédiaire (60, 61), qui contribue à générer le réseau prévisionnel (58), est déterminée en fonction des valeurs des droites de liaison de bords (34 à 57) du maillage partiel sélectionné, à la place de la valeur de la profondeur (preopACD) de la chambre antérieure (7) de l'oeil (1) du patient et / ou que, pour la valeur de la longueur (preopAL) de l'oeil (1) du patient, une deuxième droite de liaison intermédiaire (60, 61), qui contribue à générer le réseau prévisionnel (58), est déterminée en fonction des valeurs des droites de liaison de bords (34 à 57) du maillage partiel sélectionné, à la place de la valeur de la longueur (preopAL) de l'oeil (1) du patient.

**7.** Procédé selon la revendication 6,
**caractérisé en ce que**,
à la place de la paire de valeurs de la profondeur (preopACD) de la chambre antérieure (7) de l'oeil et de la longueur (preopAL) de l'oeil (1) du patient, au moins une valeur prévisionnelle de la position en profondeur postopératoire (postopeIOLE, postopeIOLE*) de la lentille intraoculaire (14, 14') dans l'oeil (1) du patient est calculée à partir d'au moins une droite de liaison intermédiaire (60, 61), en particulier respectivement une valeur prévisionnelle est calculée à partir des deux droites de liaison intermédiaires (60, 61) et que les deux valeurs prévisionnelles sont comparées et l'autre type de détermination d'une valeur prévisionnelle finale de la position en profondeur postopératoire (postopeACD, postopeIOLE) de la lentille intraoculaire (14, 14') dans l'oeil (1) du patient est fixée par rapport à la prévision préopératoire en fonction d'une différence constatée des valeurs prévisionnelles, en particulier qu'une détermination de la valeur moyenne est effectuée.

**8.** Procédé selon l'une des revendications précédentes,
**caractérisé en ce que**,
pour la détermination de la valeur postopératoire de la position en profondeur (postopeIOLE, postopeIOLE*) de la lentille intraoculaire (14, 14') dans l'oeil (1) du patient, pour une lentille intraoculaire (14, 14') prévue, avec une haptique (62, 63, 62', 63') inclinée par rapport à la verticale et / ou pour un site d'implantation prévu spécifique de la lentille intraoculaire (14, 14') dans l'oeil (1) du patient, un premier facteur de correction est pris en compte, qu'en particulier un facteur de correction est pris en compte, lequel est déterminé de manière variable en fonction de la forme de la lentille et / ou du site d'implantation dans l'oeil (1) du patient.

**9.** Procédé selon la revendication 8,
**caractérisé en ce que**
le facteur de correction (fac$_{corr}$) est déterminé par la relation fonctionnelle suivante :

$$\mathbf{\mathit{fac_{corr}} = -\ a+[(\mathit{fac_z} \cdot b)-c]},$$

moyennant quoi a se situe entre 1,3 et 1,43, b entre 0,45 et 0,65 et c entre 60 et 70, de préférence entre 64 et 66, en particulier la formule :

$$\mathbf{\mathit{fac_{corr}} = -1{,}39329+[(\mathit{fac_z} \cdot 0{,}5663)-65{,}60]}$$

est applicable et en particulier le facteur fac$_Z$ se situe entre 110 et 125 et / ou le facteur fac$_Z$ est fixé comme une fonction, en particulier une fonction linéaire, en fonction d'un paramètre de la lentille intraoculaire (14, 14'), en particulier de sa puissance de réfraction.

**10.** Procédé selon l'une des revendications précédentes,
**caractérisé en ce que**
les valeurs des profondeurs, produites pour la sélection des points d'appui principaux (18 à 33), sont déterminées par des chambres antérieures d'yeux humains par une mesure d'yeux humains et / ou les valeurs de longueurs d'yeux humains sont déterminées par une mesure d'yeux humains et / ou les valeurs de positions en profondeur postopératoires de lentilles intraoculaires dans des yeux humains sont mesurées et / ou évaluées et / ou calculées, en particulier les valeurs mesurées ne sont prises en compte que dans le cas de points d'appui principaux (18 à 33), qui sont reliés aux droites de liaison de bords (34 à 57), qui délimitent à la périphérie le réseau d'alimentation (58).

**11.** Procédé selon l'une des revendications précédentes,
**caractérisé en ce que**
les points d'appui principaux (18 à 33) sont spécifiés tels que définis par l'utilisateur.

**12.** Procédé selon l'une des revendications précédentes,
**caractérisé en ce que**
le réseau prévisionnel (58) et / ou la position en profondeur horizontale, postopératoire, déterminée et prévue avant l'opération (postopeIOLE, postopeIOLE*) de la lentille intraoculaire (14, 14') sont affichés sur une unité d'affichage d'un appareil, en particulier la position en profondeur horizontale, postopératoire, déterminée et prévue avant l'opération (postopeIOLE, postopeIOLE*) de la lentille intraoculaire (14, 14') est affichée sur une unité d'affichage d'un appareil en tant que valeur et / ou dans un oeil représenté en tant qu'image.

**13.** Procédé selon l'une des revendications précédentes,
**caractérisé en ce que**
la lentille intraoculaire (14, 14') est façonnée dans sa forme et / ou sa puissance de réfraction en fonction de la position en profondeur horizontale, postopératoire, prévue avant l'opération (postopeIOLE, postopeIOLE*) de la lentille intraoculaire (14, 14') dans l'oeil (1) du patient.

**14.** Procédé selon l'une des revendications précédentes,
**caractérisé en ce**
**qu'**un réseau prévisionnel (58), généré une fois, est corrigé en fonction d'informations reçues ultérieurement sur la situation positionnelle en profondeur réelle de la lentille intraoculaire (14, 14') dans l'oeil (1) du patient après son implantation et qu'²en particulier, les points d'appui principaux (18 à 33) correspondants sont modifiés, en particulier automatiquement.

**15.** Appareil destiné à exécuter un procédé selon l'une des revendications précédentes, lequel présente au moins un moyen pour déterminer le réseau prévisionnel (58) et comprend au moins un moyen pour déterminer la valeur prévisionnelle.

Fig.1

Fig.2

Fig.3

Fig.4

Fig.5

Fig.6

postop ACD

postope IOLE*

preop AL

Fig.7

**EP 2 705 403 B1**

**In der Beschreibung aufgeführte Patentdokumente**

- US 5282852 A
- US 20090251664 A1

- DE 69630544 T2